(19) Europäisches Patentamt
European Patent Office
Office européen des brevets

(11) Numéro de publication : **0 364 327 B1**

# (12) FASCICULE DE BREVET EUROPEEN

(45) Date de publication du fascicule du brevet : **15.01.92 Bulletin 92/03**

(51) Int. Cl.$^5$ : **C07D 215/22,** C07D 401/12, A61K 31/47

(21) Numéro de dépôt : **89402700.2**

(22) Date de dépôt : **02.10.89**

## (54) Dérivés de quinoléinone, leur préparation et leur application en thérapeutique.

(30) Priorité : **11.10.88 FR 8813324**

(43) Date de publication de la demande : **18.04.90 Bulletin 90/16**

(45) Mention de la délivrance du brevet : **15.01.92 Bulletin 92/03**

(84) Etats contractants désignés : **AT BE CH DE ES FR GB GR IT LI LU NL SE**

(56) Documents cités :
**GB-A- 2 017 701**
**GB-A- 2 071 094**

(73) Titulaire : **SYNTHELABO**
**58 rue de la Glacière**
**F-75013 Paris (FR)**

(72) Inventeur : **Manoury, Philippe**
**L'Orée de Verrières, 38, Avenue des Vaupépins**
**F-91370 Verrières le Buisson (FR)**
Inventeur : **Obitz, Daniel**
**64, rue Velpeau**
**F-92160 Antony (FR)**
Inventeur : **Peynot, Michel**
**2, rue des Marguerites**
**F-94240 L'Hay les Roses (FR)**
Inventeur : **Frost, Jonathan**
**23, Route de Montjean**
**F-91320 Wissous (FR)**

(74) Mandataire : **Thouret-Lemaitre, Elisabeth et al**
**SYNTHELABO Service Brevets 22, avenue Galilée**
**F-92352 LE PLESSIS ROBINSON CEDEX (FR)**

EP 0 364 327 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet des dérivés de quinoléinone, leur préparation et leur application en thérapeutique.

Les composés de l'invention répondent à la formule (I) donnée en annexe, dans laquelle $R_1$ et $R_2$ sont chacun, indépendamment l'un de l'autre, un atome d'hydrogène ou d'halogène ou un radical $(C_{1-4})$alkyle, $R_3$ est un atome d'hydrogène ou un radical $(C_{1-4})$alkyle, $R_4$ est un radical naphtalényle, tétrahydronaphtalényle, benzyle, phényle, pyridyle, isoquinolyle ou benzoyle, ces radicaux pouvant porter un substituant tel que méthoxy, chlore, fluor ou $(C_{3-5})$ cycloalkyle, X et Y sont chacun un atome d'hydrogène ou forment ensemble une liaison.

Lorsque X et Y sont chacun un atome d'hydrogène les composés (I) comportent un ou plusieurs atomes de carbone asymétriques. Les diastéréoisomères et les énantiomères de ces composés font partie de l'invention.

Les sels d'addition aux acides pharmaceutiquement acceptables que forment les composés (I) font également partie de l'invention.

Les composés préférés de l'invention sont ceux pour lesquels

$R_1$ et $R_2$ sont chacun, indépendamment l'un de l'autre, un atome d'hydrogène ou de fluor ou le radical méthyle,

$R_3$ est un atome d'hydrogène ou le radical méthyle ou éthyle,

$R_4$ est le radical naphtalényle ou tétrahydronaphtalényle, éventuellement substitué,

X et Y représentent une liaison ou chacun un atome d'hydrogène.

GB-A-2017701 et GB-A-2071094 décrivent des dérivés de carbostyril leur application comme antitistaminiques et leur activité sur le SNC.

Selon l'invention, on peut préparer les composés (I) selon les schémas réactionnels donnés en annexes 1, 2 et 3.

Lorsque X et Y représentent ensemble une liaison, on prépare les composés suivant le schéma réactionnel de l'annexe 1 : on fait réagir une phénylamine (II) avec l'(acétyloxy)-4 2H,3H-pyrannedione-2,6 et on cyclise le composé obtenu (III) en acide (IV) que l'on transforme en ester (V) puis en alcool (VI) et enfin en chlorure (VII) que l'on condense avec une $R_4$-1 pipérazine pour obtenir le composé (I), que l'on alkyle, si on le souhaite, avec un composé $R_3X$ (X = groupe labile).

Lorsque X et Y sont chacun un atome d'hydrogène on peut préparer les composés (I) par hydrogénation du noyau oxoquinoléinyle à un stade quelconque de la synthèse, même au stade du produit final.

Le schéma réactionnel de l'annexe 2 consiste à hydrogéner l'ester (V) en ester (VIII) que l'on réduit en alcool (IX) (ce dernier peut aussi être obtenu par hydrogénation de l'alcool (VI)). On transforme ce composé (IX) en composé chloré (X) que l'on condense avec une $R_4$-1 pipérazine pour obtenir les composés (I) dans lesquels $R_3$ = H et que l'on peut éventuellement alkyler avec un composé $R_3X$.

On prépare les énantiomères des composés (I), dans lesquels X et Y sont chacun un atome d'hydrogène, selon le schéma réactionnel donné en annexe 3, par dédoublement de l'acide (XI) (obtenu par saponifaction de l'ester (VIII)), suivant le procédé classique de séparation des sels diastéréoisomères résultant de la salification de cet acide par des amines optiquement actives telles que la L-tyrosine hydrazide ou la (naphtyl-1)-1 éthylamine. On réduit ensuite les acides dextrogyre ou lévogyre (XI) via leurs anhydrides mixtes en alcools (IX) (de même signe), lesquels sont ensuite transformés en composés chlorés (X) dextroygre et lévogyre. On condense ces derniers avec une $R_4$-1 pipérazine de manière à obtenir les composés (I) dextrogyre et lévogyre dans lesquels $R_3$ est H, et que l'on peut éventuellement alkyler.

Les exemples suivants illustrent l'invention ; les analyses et les spectres IR et RMN confirment la structure des composés.

Exemple 1.

Méthyl-6 [[(naphtalényl-1)-4 pipérazinyl-1]-2 éthyl]-4 1H-quinoléinone-2 (schéma annexe 1).

1.1. Acide méthyl-6 oxo-2 dihydro-1,2 quinoléine-4-acétique.

a) A une solution de 31 g (0,29 mol) de méthyl-4 aniline dans 100 ml d'acide acétique on ajoute assez rapidement, en agitant 50 g (0,29 mol) d'(acétyloxy)-4 2H,3H-pyrannedione-2,6 (préparée selon E.G. Frandsen et N. Jacobsen J. Chem. Soc. Perkin, I 933-6 (1978).

Après 30 minutes d'agitation à température ambiante on ajoute 800 ml d'eau et laisse agiter 1 h. Le solide est alors essoré, lavé à l'eau et séché à 50° pendant 24 h. On obtient 68,4 g (85%) d'un solide fondant à 141-2°C.

b) A 500 ml d'acide sulfurique (95-97%), on ajoute par petites portions 90 g (0,325 mol) de l'anilide précédent puis on chauffe à 100° tout en agitant pendant 2 h. Après refroidissement on verse le milieu réactionnel dans 500 g de glace et 200 ml d'eau, agite et essore le solide que l'on lave 3 fois à l'eau avant de le sécher 8 h à 60°. On recueille 60 g (85%) d'un solide fondant à 253-5°C.

1.2. Méthyl-6 oxo-2 dihydro-1,2 quinoléine-4-acétate de méthyle.

On ajoute, goutte à goutte, en 1 h environ, 95 g (0,8 mol) de chlorure de thionyle à une suspension agitée de 60 g (0,276 mol) de l'acide précédent dans 1 litre de méthanol puis laisse agiter 4 h. Après concentration à 500 ml et refroidissement dans la glace le solide est essoré, lavé au méthanol puis à l'éther et séché à 60° pendant 8 h. On obtient 57 g (89%) d'un solide fondant à 219-20°C.

1.3. (Hydroxy-2 éthyl)-4 méthyl-6 1H-quinoléinone-2.

A une suspension de 45,6 g (1,2 mol) d'hydrure de lithium et d'aluminium dans 1,5 1 de tétrahydrofuranne sec, on ajoute par portions sous argon, 56,5 g (0,244 mol) de l'ester précédent en refroidissant par de la glace pour maintenir la température du milieu inférieure à 30°. Après l'addition, on laisse agiter 4 h à température ambiante. On hydrolyse le milieu réactionnel par addition très lente de 200 ml d'eau, laisse au repos une nuit, puis on le verse sur un mélange de 1 kg de glace et 200 ml d'acide sulfurique ; le THF est alors éliminé par évaporation sous pression réduite. La suspension du composé attendu dans l'eau acidulée est essorée, le solide est lavé par l'eau puis séché. Il est ensuite lavé en suspension dans 500 ml d'éthanol à reflux pendant 1 h. Après refroidissement on essore, lave à l'éther et sèche à 60° 35,5 g (72%) d'un solide fondant à 252-5°C.

1.4. (Chloro-2 éthyl)-4 méthyl-6 1H-quinoléinone-2.

A une suspension de 15 g (0,074 mol) du carbinol précédent dans 700 ml de chloroforme, on additionne en agitant 29 ml (0,4 mol) de chlorure de thionyle et 10 gouttes de pyridine. Il apparaît une huile qui cristallise peu à peu à température ambiante. On chauffe la suspension au reflux du solvant jusqu'à sa solubilisation totale (environ 4 h). En refroidissant et en agitant, on ajoute lentement et avec précaution 400 ml d'eau au milieu réactionnel et laisse encore agiter 30 minutes. Le produit cristallisé obtenu est essoré et la phase chloroformique du filtrat est décantée, lavée à l'eau et séchée. Après évaporation du solvant, on triture le résidu dans l'alcool et obtient un second jet de solide. On réunit les deux fractions solides et on les recristallise dans l'éthanol. On obtient 10,25 g (62,5%) d'un solide fondant à 215-6°C.

1.5. Méthyl-6 [[(naphtalényl-1)-4 pipérazinyl-1]-2 éthyl]-4 1H-quinoléinone-2.

On chauffe 30 minutes au reflux une solution de 1,5 g (0,0068 mol) du dérivé chloré précédent et de 3,2 g (0,015 mol) de (naphtalényl-1)-1 pipérazine (décrite par Prelog et Blazek, Collect. Czech. Chem. Comm. 6,211-5 (1934)) dans 50 ml d'alcool puis évapore l'alcool à siccité. Le résidu pâteux est chauffé 1 h à 120-30°. Le mélange de sel et de base résultant est une huile que l'on triture dans une solution aqueuse saturée de carbonate de sodium puis on en évapore l'eau à siccité. A cause de leur insolubilité dans les solvants usuels les bases organiques sont extraites par un mélange chlorure de méthylène-méthanol (50-50), la suspension résultante est filtrée et le filtrat concentré. L'huile résiduelle est purifiée sur colonne de silice en éluant avec le mélange chlorure de méthylène-méthanol (95-5). La fraction obtenue est recristallisée du méthylcellosolve et le solide isolé est séché à 100° sous pression réduite. On obtient 1,9 g (71%) d'un solide fondant à 241-2°C.

Exemple 2. Fluoro-7 [[(méthoxy-7 naphtalényl-1)-4 pipérazinyl-1]-2 éthyl]-4 1H-quinoléinone-2.

2.1. Acide fluoro-7 oxo-2 dihydro-1,2 quinoléine-4-acétique.

Le composé est préparé selon la technique décrite en 1.1. à partir de la fluoro-3 aniline. Il contient 17% d'acide fluoro-5 oxo-2 dihydro-1,2 quinoléine-4-acétique. Ce mélange fond à 224-5°C.

2.2. Fluoro-7 oxo-2 dihydro-1,2 quinoléine-4-acétate de méthyle.

Le composé est obtenu selon la technique décrite en 1-2. Il est pur à 92% et contient 8% de fluoro-5 oxo-2 dihydro-1,2 quinoléine-4-acétate de méthyle. Il fond à 255-6°C.

2.3. Fluoro-7 (hydroxy-2 éthyl)-4 1H-quinoléinone-2.

Préparé avec un rendement de 70,4% selon le mode opératoire décrit en 1-3 ce produit est pur à 97,5% et fond à 221-2°C.

2.4. (Chloro-2 éthyl)-4 fluoro-7 1H-quinoléinone-2.

Obtenu selon la technique décrite en 1-4 avec un rendement de 34% ce produit fond à 190-1°C.

2.5. Fluoro-7 [[(méthoxy-7 naphtalényl-1)-4 pipérazinyl-1]-2 éthyl]-4 1H-quinoléinone-2.

Préparé (57%) à partir de la (méthoxy-7 naphtalényl-1)-1 pipérazine décrite dans le brevet français n° 88.10481 selon la technique décrite en 1.5. Ce composé fond à 250-2°C.

Exemple 3. (±)[[(Méthoxy-7 naphtalényl-1)-4 pipérazinyl-1]-2 éthyl]-4 dihydro-3,4 1H-quinoléinone-2 (schéma annexe 2).

3.1.Acide acétyloxy-3 oxo-5 phénylamino-5 pentène-2 oïque.

A une suspension agitée de 120 g (0,705 mol) d'acétyloxy-4 2H,3H-pyrannedione-2,6 dans 250 ml d'acide acétique, on ajoute 65,7 g (0,705 mol) d'aniline. On agite le milieu réactionnel $1^h$ à la température ambiante puis on le dilue par addition de 500 ml d'eau. Le solide est essoré, lavé à l'eau et séché à l'étuve (50°C) pendant 24 h. On obtient 150 g (81%) du composé attendu qui fond à 125-6°C.

Ce composé est décrit par E.G. Frandsen et N. Jacobsen (référence citée en 1.1).

3.2. Acide oxo-2 dihydro-1,2 quinoléine-4-acétique.

On verse, par petites portions, 150 g (0,57 mol) de l'acide acétyloxy-3 oxo-5 phénylamino-5 pentène-2 oïque dans 500 ml d'acide sulfurique à 95-97% en agitant, puis on chauffe le milieu réactionnel à 100°C pendant 2 h. Après refroidissement on verse cette solution dans un mélange de 1 kg de glace et 500 ml d'eau en agitant. On essore le solide ainsi obtenu. On le lave avec 3 fois 100 ml d'eau et le sèche pendant 8 h à 60°C. On obtient 60,5 g (52%) de l'acide oxo-2 dihydro-1,2 quinoléine-4-acétique fondant à 213-5°C.

Ce composé est décrit par E. Besthorn et E. Garben, Chem. Ber. (1900), 33, 3439.

3.3 Oxo-2 dihydro-1,2 quinoléine-4-acétate de méthyle.

On ajoute, goutte à goutte, en 1 h environ, 95 g (0,8 mol) de chlorure de thionyle à une suspension agitée de 60 g (0,295 mol) d'acide oxo-2 dihydro-1,2 quinoléine-4-acétique dans 1l de méthanol. On abandonne une nuit. On chasse le solvant et on dissout le résidu obtenu dans 300 ml de méthanol bouillant. On ajoute au mélange, après refroidissement, 300 ml d'éther. Le solide est essoré puis recristallisé dans du méthanol. On obtient 51,5 g (80%) d'oxo-2 dihydro-1,2-quinoléine-4-acétate de méthyle fondant à 209-210°C.

Ce produit est décrit par M. Uchida, F. Tabusa, M. Komatsu, S. Morita, T. Kanbe et K. Nakagawa. Chem. Pharm. Bull. (1985), 33 3775.

3.4.(±)-oxo-2 tétrahydro-1,2,3,4 quinoléine-4-acétate de méthyle

Dans un appareil de Parr on soumet à l'hydrogénation 50 g (0,23 mol) d'oxo-2 dihydro-1,2 quinoleine-4-acétate de méthyle en solution dans 1 l de méthanol en présence de 2 g d'oxyde de platine d'Adams sous 40 à 45 psi et à 60°C pendant environ 1 h. Après filtration du catalyseur et évaporation du solvant, le résidu huileux est chromatrographié sur silice. L'élution avec un mélange dichlorométhane/acétone (95 : 5) fournit une huile qui est solubilisée dans 250 ml de toluène bouillant. Après refroidissement, cette solution est diluée par 250 ml d'éther de pétrole. Le produit cristallin ainsi obtenu est essoré, lavé et séché. On obtient 36,8 g (73%) de (±)-oxo-2 tétrahydro-1,2,3,4 quinoléine-4-acétate de méthyle fondant à 115-6°C.

3.5. (±)-(Hydroxy-2 éthyl)-4 dihydro-3,4 1H-quinoléinone-2.

A une suspension de 2,65 g (0,07 mol) d'hydrure de lithium et d'aluminium dans 250 ml d'éther sec, tout en agitant sous atmosphère inerte, on verse, goutte à goutte, une solution de 11 g (0,05 mol) de (±)-oxo-2 tétrahydro-1,2,3,4 quinoléine-4-acétate de méthyle dans 50 ml de dioxanne. Le mélange est ensuite chauffé pendant 4h au reflux. Après refroidissement, la réaction est hydrolysée par lente addition de 10 ml d'eau avec agitation énergique. La suspension est filtrée et le solide minéral est lavé par 20 ml de dioxanne. Le filtrat est évaporé et l'huile obtenue est triturée dans l'éther, ce qui la fait cristalliser. Le solide est recristallisé dans de l'acétate d'éthyle. On obtient 5,3 g (55%) de (±)-(hydroxy-2 éthyl)-4 dihydro-3,4 1H-quinoléinone-2 fondant à 119-120°C.

3.6. (±)-(chloro-2 éthyl)-4 dihydro-3,4 1H-quinoléinone-2.

On chauffe à reflux, pendant 4 h, un mélange de 5,2 g (0,027 mol) de (±)-(hydroxy-2 éthyl)-4 dihydro-3,4 1H-quinoléinone-2, 15 g (0,126 mol) de chlorure de thionyle, 200 ml de chloroforme sec et 10 gouttes de pyridine. Après refroidissement, on ajoute goutte à goutte 50 ml d'eau et agite 30 minutes. La phase organique est décantée, séchée sur sulfate de magnésium et concentrée sous vide. Le résidu est chromatographié sur colonne de silice. L'élution avec un mélange dichlorométhane/acétone (90/10) fournit une huile qui est cristallisée dans du cyclohexane. On obtient 5 g (88%) de (±)-chloro-2 éthyl)-4 dihydro-3,4 1H-quinoléinone-2 fondant à 112-3°C.

3.7. (±)-[[(méthoxy-7 naphtalényl-1)-4 pipérazinyl-1]-2 éthyl]-4 dihydro-3,4 1H-quinoléinone-2.

On mélange, intimement, 1,5 g (7,15 mmol) de (±)-(chloro-2 éthyl)-4 dihydro-3,4 1H-quinoléinone-2 avec 3,65 g (15 mmol) de (méthoxy-7 naphtalényl-1)-1 pipérazine et on chauffe à 130°C pendant 1 h tout en agitant. Le résidu refroidi est repris entre 25 ml de soude 2N et 2 fois 50 ml de dichlorométhane. L'extrait organique est lavé à l'eau, séché sur sulfate de magnésium et concentré. Le résidu est purifié par élution avec un mélange dichlorométhane/méthanol (95/5) sur colonne de silice. La fraction purifiée est cristallisée dans l'éther et ce solide est essoré et séché. On obtient 2,6 g (87,5%) de (±)-[[(méthoxy-7 naphtalényl-1)-4 pipérazinyl-1]-2 éthyl]-4 dihydro-3,4 1H-quinoléinone-2 fondant à 177-8°C.

3.8. Sesquifumarate de (±)-[[(méthoxy-7 naphtalényl-1)-4 pipérazinyl-1]-2 éthyl]-4 dihydro-3,4 1H-quinoléinone-2 (base/acide 2/3).

A une solution de 2,5 g (6,02 mmol) de la base précédente dans 100 ml d'éthanol bouillant on ajoute 1,06 g (9,1 mmol) d'acide fumarique. Quand tout est solubilisé à chaud on laisse refroidir lentement la solution. Le sel cristallisé est essoré, lavé à l'alcool puis séché sous pression réduite à 100°C. On obtient 2,9 g (82%) du sesquifumarate de (±)-[[(méthoxy-7 naphtalényl-1)-4 pipérazinyl-1]-2 éthyl]-4 dihydro-3,4 1H-quinoléinone-2 (base/acide 2/3) fondant à 208-9°C.

On peut utiliser le procédé de préparation de l'annexe 2 pour obtenir le composé 3.5.

On prépare l'(hydroxy-2 éthyl)-4 1H-quinoléinone-2 (composé 3.9) à partir de l'oxo-2 dihydro-1,2 quinoléine-4-acétate de méthyle (préparé en 3.3) selon le mode opératoire décrit en 1.3. C'est un solide fondant à 213-5°C. On effectue une hydrogénation catalytique au platine d'Adams de ce composé pour obtenir la (±)-(hydroxy-2 éthyl)-4 dihydro-3,4 1H-quinoléinone-2.

Exemple 4. [[(Naphtalényl-1)-4 pipérazinyl-1]-2 éthyl]-4 méthyl-1 quinoléinone-2.

4.1. (Chloro-2 éthyl)-4 1H-quinoléinone-2.

Ce composé est préparé à partir du carbinol décrit en 3.9. selon la technique décrite en 1.4. On obtient 62,5% d'un solide fondant à 212°. Ce composé est décrit dans le brevet Ger. Offen 3324034 (5/1/84) de Otsuka Pharm. Co. Ltd avec un point de fusion de 186-7°C.

4.2. [[(Naphtalényl-1)-4 pipérazinyl-1]-2 éthyl]-4 1H-quinoléinone-2.

Ce composé est obtenu avec un rendement de 50% en suivant la technique décrite en 1.5. et fond à 230-232°C.

4.3. [[(Naphtalényl-1)-4 pipérazinyl-1]-2 éthyl]-4 méthyl-1 quinoléinone-2.

On chauffe 1 h à 60-70° sous argon une suspension de 0,312 g d'hydrure de sodium (lavé au pentane) dans 50 ml de diméthylsulfoxyde. A cette solution refroidie on additionne à température ambiante 2,5 g (6,5

mmol) du composé précédent et laisse agiter 30 minutes puis refroidit à 10° et additionne une solution de 1 g d'iodure de méthyle dans 10 ml de DMSO. Après 12 h de réaction à température ordinaire on ajoute 200 ml d'eau et essore un solide gommeux lequel est élué sur une colonne de silice par le mélange chlorure de méthylèneméthanol (95-5). La fraction purifiée fondant trop bas on en prépare le monofumarate dans l'éthanol. Après cristallisation, essorage et lavage à l'éther on obtient 2,2 g (66%) d'un solide fondant à 208-9°C.

Exemple 5.

(+)-[[(méthoxy-7 naphtalényl-1)-4 pipérazinyl-1]-2 éthyl]-4 dihydro-3,4 1H-quinoléinone-2.

5.1. Acide (±)-oxo-2 tétrahydro-1,2,3,4 quinoléine-4-acétique.

A une suspension de 58 g (0,265 mol) de l'ester obtenu en 3.4 dans 500 ml d'une solution méthanol/eau (50/50) on ajoute, en agitant, 11 g de NaOH à 97% (0,27 mol). Après 1 h d'agitation à la température ambiante, la solution homogène est laissée au repos une nuit. Après évaporation du méthanol, on ajoute 250 ml d'eau, puis HCl 6N, goutte à goutte, en agitant, jusqu'à pH = 1.

L'acide précipité est filtré après 1 h d'agitation à la température ambiante. On le lave 3 fois à l'eau, et on le sèche à 100°C sous vide. On obtient 52,5 g (96,5%) d'un solide fondant à 183 4°C.

5.2. Sel de L-tyrosine-hydrazide de l'acide (+)-oxo-2 tétrahydro-1,2,3,4 quinoléine-4-acétique.

A une solution bouillante de 19,5 g (0,1 mol) de L-tyrosine hydrazide dans 1l de méthanol/eau (90/10), on ajoute 20,5 g (0,1 mol) de l'acide obtenu en 5.1.

On agite 4 h en maintenant le mélange réactionnel à 30-40°C, puis on le laisse une nuit au repos à la température ambiante. On filtre, lave par le méthanol et sèche le solide. Après 2 recristallisations dans un mélange $CH_3OH/H_2O$ (90/10) on obtient 8,25 g (20,6%) d'un composé fondant à 209-210°C. $[\alpha]_D^{24}$ = + 32,5° (c = 1, DMF)

5.3. Acide (+)-oxo-2 tétrahydro-1,2,3,4 quinoléine-4-acétique.

On chauffe jusqu'à dissolution, une suspension de 8 g de sel de L-tyrosine hydrazide obtenu en 5.2 dans 250 ml d'eau distillée. On ajoute, en agitant, 5 ml d'acide chlorhydrique concentré (36-38%) et on laisse cristalliser l'acide dextrogyre à froid (5-10°C environ).

On filtre, lave 3 fois par un minimum d'eau glacée et sèche 8 h à 100°C sous vide. On obtient 3,8 g (93%) d'un solide fondant à 154-5°C.
$[\alpha]_D^{20}$ = + 63,2° (c = 1, DMF).

5.4. (+)-(Hydroxy-2 éthyl)-4 dihydro-3,4 1H-quinoléinone-2.

On refroidit à – 10°C une solution de 4,1 g (0,02 mol) de l'acide obtenu en 5.3 et de 2,02 g (0,02 mol), de triéthylamine dans 100 ml de THF anhydre, sous agitation et en atmosphère d'argon sec.

On ajoute goutte à goutte, en 15 mn, une solution de 2,17 g (0,02 mol) de chloroformiate d'éthyle dans 10 ml de THF sec. Après 1 h d'agitation à – 5°C, – 7°C, on ajoute par portions en 5 mn environ, 1,5 g (0,04 mol) de borohydrure de sodium. On laisse revenir le mélange réactionnel à la température ambiante, et on agite encore 30 mn. On ajoute, goutte à goutte, 20 ml d'eau (en 30 mn environ). On décante la phase organique et on extrait 3 fois au dichlorométhane la phase aqueuse résiduelle contenant un minéral insoluble. On sèche les extraits sur sulfate de magnésium, évapore le solvant et purifie sur colonne de silice avec l'éluant dichlorométhane/méthanol (95/5).

La fraction purifiée est évaporée. On obtient 2,7 g (70,7%) d'une huile qui cristallise par trituration dans l'éther.
F = 105-6°C.
$[\alpha]_D^{20}$ = + 44,5° (c = 1, DMF).

5.5. (+)-(Chloro-2 éthyl)-4 dihydro-3,4 1H-quinoléinone-2.

On chauffe 4 h à la température du reflux, sous azote sec, 2,5 g (13,07 mmol) du composé 5.4, 5 ml (69 mmol) de chlorure de thionyle, 5 gouttes de pyridine et 100 ml de chloroforme sec. A froid on ajoute, goutte à goutte, sous agitation, 30 ml d'eau, et on décante.

L'extrait chloroformique, séché sur sulfate de magnésium et évaporé, est ensuite purifié sur colonne de silice avec les eluants dichlorométhane/acétone (95/5 puis 90/10).

Les fractions purifiées donnent une huile qui cristallise par trituration dans du cyclohexane. Après filtration, lavage par le pentane et séchage 8 h à 60°C sous vide on obtient 1,3 g (47,5%) d'un composé qui fond à 110-111°C.

$[\alpha]_D^{20}$ = + 77,1° (c = 1, DMF).

5.6. (+)[[(Méthoxy-7 naphtalényl-1)-4 pipérazinyl-1]-2 éthyl]-4 dihydro-3,4 1H-quinoléinone-2.

On chauffe 4 h, à la température du reflux, 1,2 g (5,72 mmol) de dérivé 5.5, 1,45 g (6 mmol) de (méthoxy-7 naphtalényl-1)-1 pipérazine, 20 ml de méthylisobutylcétone (MIBC) et quelques cristaux d'iodure de sodium. On ajoute 0,32 g (3 mmol) de carbonate de sodium et chauffe le mélange à la température du reflux pendant 8 h.

On rajoute 0,32 g (3 mmol) de carbonate de sodium et laisse le mélange au reflux encore 8 h. Après addition de 1 ml d'eau et 4 h de reflux, on évapore la MIBC, et reprend le résidu par 50 ml d'eau. On extrait la base par le dichlorométhane. L'extrait séché sur sulfate de magnésium et évaporé, est purifié sur colonne de silice avec l'éluant dichlorométhane/ méthanol (95/5).

La fraction purifiée cristallise dans l'éther et le solide est recristallisé dans de l'alcool isopropylique. On obtient 1,55 g (65%) d'un solide fondant à 149-50°C.

$[\alpha]_D^{25}$ = + 50,5°(C = 1, DMF).

Exemple 6. (–)-[[(Méthoxy-7 naphtalényl-1)-4 pipérazinyl-1]-2 éthyl]-4 dihydro-3,4 1H-quinoléinone-2.

6.1. Sel de (+)-(naphtalényl-1)-1 éthylamine et d'acide (–)-oxo-2 tétrahydro-1,2,3,4 quinoléine-4-acétique.

On évapore à siccité les solutions mères des 3 recristallisations du sel de L-tyrosine-hydrazide et d'acide (±)oxo-2 tétrahydro-1,2,3,4 quinoléine-4-acétique (v. exemple 5.2.) On met en solution le résidu dans 200 ml d'eau à 50-60°C et on ajoute 20 ml d'acide chlorhydrique concentré.

L'acide enrichi en énantiomère lévogyre cristallise à froid et fond à 178-180°C.

On met en solution 13,5 g (65,8 mmol) de cet acide enrichi et 11,3 g (66 mmol) de (R)(+)-(naphtalényl-1)-1 éthylamine dans 350 ml d'alcool isopropylique bouillant.

A la température ambiante, après 4 h d'agitation, on filtre le mélange réactionnel et lave le solide à l'éther.

Après 2 recristallisations dans un mélange alcool isopropylique-eau (95/5), on obtient 6,1 g (24,6%) d'un composé fondant à 192-3°C.

$[\alpha]_D^{20}$ = – 1,75° (c = 1, DMF).

6.2. Acide (–)oxo-2 tétrahydro-1,2,3,4 quinoléine-4-acétique.

On chauffe jusqu'à dissolution, une suspension de 6 g (15,9 mmol) du sel précédent 6.1 dans 100 ml d'eau distillée et 5 ml d'acide chlorhydrique concentré (36-8%).

On laisse cristalliser le produit à froid (5-10°C environ). On filtre et lave le solide à l'eau glacée. On le sèche 8 h à 100°C sous vide et obtient 2,95 g (90,5%) d'un composé fondant à 154-5°C.

$[\alpha]_D^{20}$ = – 62,7° (c = 1, DMF).

6.3. (–)-(Hydroxy-2 éthyl)-4 dihydro-3,4 1H-quinoléinone-2.

On refroidit à – 10°C une solution de 2,85 g (13,9 mmol) d'acide (–)oxo-2 tétrahydro-1,2,3,4 quinoléine-4-acétique et de 1,42 g (14 mmol) de triéthylamine dans 70 ml de THF sec, en agitant, sous argon sec.

On ajoute goutte à goutte, en 20 mn, une solution de 1,52 g (14 mmol) de chloroformiate d'éthyle dans 10 ml de THF sec. Après 1 h d'agitation à – 5°C, on ajoute par portions, en 5 mn, 1,13 g (30 mmol) de borohydrure de sodium.

On laisse revenir le mélange réactionnel à la température ambiante en agitant encore 30 mn, puis on ajoute, goutte à goutte, 15 ml d'eau. On évapore le milieu et on extrait le résidu au dichlorométhane (3 × 50 ml). L'extrait séché sur sulfate de magnésium et évaporé, est ensuite purifié sur colonne de silice avec l'éluant dichlorométhane/méthanol (95/5). La fraction purifiée donne une huile à l'évaporation. Le produit cristallise par trituration dans de l'éther. On obtient 1,9 g (71,4%) d'un solide fondant à 105-6°C.

$[\alpha]_D^{25}$ = – 44,7° (c = 1, DMF).

6.4. (–)-(Chloro-2 éthyl)-4 dihydro-3,4 1H-quinoléinone-2.

On chauffe, 4 h, au reflux, sous azote sec, 1,8 g (9,4 mmol) du composé 6.3, 3,4 ml de chlorure de thionyle ( ~47 mmol), 70 ml de chloroforme sec et 5 gouttes de pyridine.

A froid, on ajoute goutte à goutte, sous agitation vigoureuse, un excès d'eau et on décante. L'extrait chloroformique est séché sur sulfate de magnésium, puis évaporé et purifié sur colonne de silice avec l'éluant dichlorométhane/acétone (90/10). La fraction purifiée donne après dissolution à chaud une huile qui cristallise dans du cyclohexane. On obtient 1,55 g (79%) d'un solide fondant à 109-10°C.
$[\alpha]_D^{20} = -76,8°$ (c = 1, DMF).

6.5.(–)[[(Méthoxy-7 naphtalényl-1)-4 pipérazinyl-1]-2 éthyl]-4 dihydro-3,4 1H-quinoléinone-2.

On chauffe 4 h à la température du reflux, 1,45 g (6,9 mmol) du dérivé chloré 6.4, 1,7 g (7 mmol) de (méthoxy-7 naphtalényl-1)-1 pipérazine, quelques cristaux d'iodure de sodium et 20 ml de méthylisobutylcétone.

On ajoute 0,37 g (3 mmol) de carbonate de sodium et porte le mélange réactionnel à la température du reflux pendant 8 h. On rajoute 0,37 g de carbonate de sodium et laisse encore 8 h au reflux. Après addition de 1 ml d'eau et 4 h de reflux, on évapore la méthylisobutylcétone, et reprend le résidu par 50 ml d'eau. On extrait la base par le dichlorométhane. L'extrait séché sur sulfate de magnésium et évaporé, est purifié sur colonne de silice avec l'éluant dichlorométhane/méthanol (95/5). La fraction purifiée cristallise dans de l'éther et on recristallise le solide dans l'alcool isopropylique. Après filtration et séchage on obtient 1,8 g (68%) de cristaux fondant à 149-50°C.
$[\alpha]_D^{25} = -49,8°$ (c = 1, DMF).

Tableau

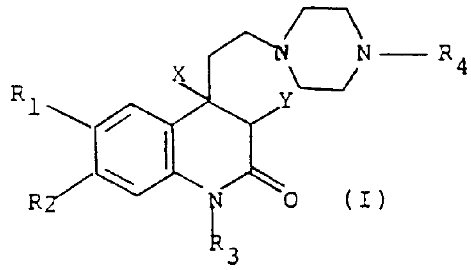

(I)

| Composé | $R_1$ | $R_2$ | $R_3$ | X,Y | $R_4$ | F(°C)(sel) |
|---|---|---|---|---|---|---|
| 1 | H | H | H | liaison | 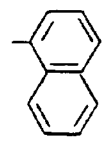 | 230-2 |
| 2 | H | F | H | liaison | 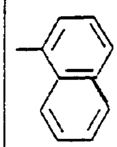 | 226-7 |
| 3 | H | H | H | liaison | 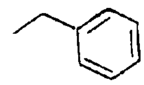 | 167-8 |
| 4 | H | H | H | liaison | 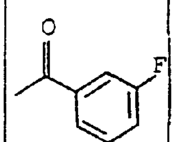 | 172-4 |
| 5 | $CH_3$ | H | H | liaison | 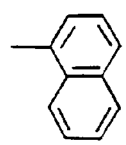 | 241-2 |
| 6 | $CH_3$ | H | H | liaison | 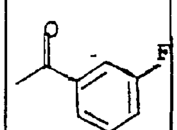 | 254-6 |
| 7 | $CH_3$ | H | H | liaison | 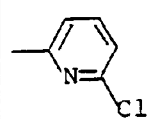 | 227-9 (base) 204-5 (maléate) |
| 8 | H | H | H | liaison | 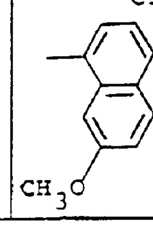 | 234-5 |

Tableau (suite)

| Composé | $R_1$ | $R_2$ | $R_3$ | X,Y | $R_4$ | F(°C)(sel) |
|---|---|---|---|---|---|---|
| 9 | H | F | H | liaison | 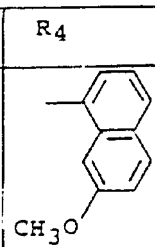 | 250-2 |
| 10 | H | H | H | liaison | 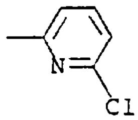 | 236-8(base)<br>206-7<br>(maléate) |
| 11 | H | H | H | H,H | 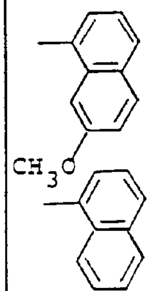 | 177-8(base)<br>208-9* |
| 12 | H | H | $CH_3$ | liaison | | 208-9<br>(fumarate) |
| 13 | H | H | $CH_2CH_3$ | liaison | 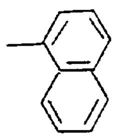 | 197-8<br>(fumarate) |
| 14 | H | H | H | liaison | 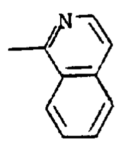 | 226-7 |
| 15 | H | H | H | liaison | 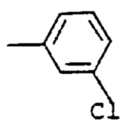 | 215-6 |
| 16 | $CH_3$ | H | H | H,H | 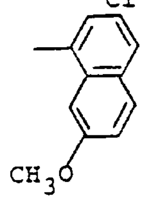 | 181-2 (base)<br>226-7<br>(fumarate) |

* sesquifumarate

Tableau (suite)

| Composé | $R_1$ | $R_2$ | $R_3$ | X,Y | $R_4$ | F(°C)(sel) |
|---|---|---|---|---|---|---|
| 17 | H | H | H | liaison | 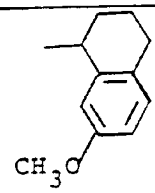 | 180-1 |
| 18 | H | H | H | H,H | 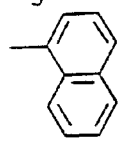 | 175-6 (base) 237-8 (fumarate) |
| 19 | $CH_3$ | H | H | H,H | 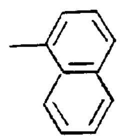 | 181-2 (base) 203-5 (fumarate) |
| 20 | H | H | H | H,H | 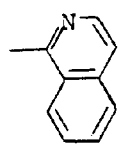 | 167-8 (base) 209-10 (fumarate) |
| 21 | $CH_3$ | H | H | H,H | 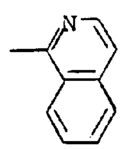 | 168-9 (base) 200-2 (fumarate) |
| 22 | H | H | $CH_3$ | H,H | 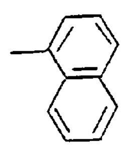 | 135-6 (base) 192-3 (fumarate) |
| 23 | H | H | H | H,H | 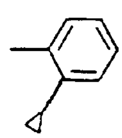 | 101-2 (base) 228-9 (fumarate) |

Tableau (fin)

| Composé | $R_1$ | $R_2$ | $R_3$ | X,Y | $R_4$ | F(°C)(sel) |
|---|---|---|---|---|---|---|
| 24 | H | H | $CH_3$ | H,H | 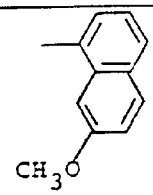 | 226-227 (oxalate) |
| 25 | H | H | H | liaison | 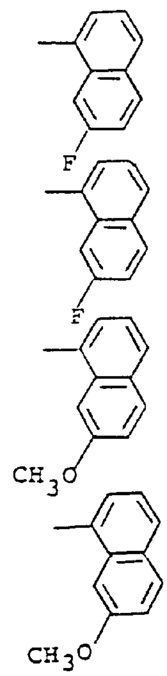 | 225-6 |
| 26 | H | H | H | H,H | | 197-8 (base) 225-6 (fumarate) |
| 27 (+) | H | H | H | H,H | | 149-50 |
| 28 (-) | H | H | H | H,H | | 149-50 |
| 29 | H | F | H | H,H | 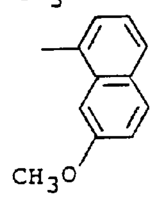 | 165-6 |

Les composés de l'invention ont été soumis à une série d'essais pharmacologiques qui ont mis en évidence leur intérêt comme substances à activités thérapeutiques.

Ainsi ils ont fait l'objet d'une étude quant à leur affinité pour les récepteurs sérotoninergiques du type 5-$HT_{1A}$. Les composés déplacent dans l'hippocampe du rat un ligand spécifique marqué, la [$^3$H]-hydroxy-8 dipropylamino-2 tétraline (désignée ci-après par "[$^3$H]-8-OH-DPAT") décrite par Gozlan et coll., Nature, (1983), 305, 140-142.

Les animaux utilisés sont des rats mâles Sprague-Dawley de 160 à 200 g. Après décapitation on en prélève le cerveau et on excise l'hippocampe. On broie le tissu dans un appareil Ultra-Turrax Polytron pendant 30 s à la moitié de la vitesse maximale dans 10 volumes de tampon Tris 50 mM d'un pH ajusté à 7, 4 avec de l'acide chlorhydrique (soit 100 mg de tissu frais par ml). On lave les tissus homogénéisés trois fois à 4°C, en les centrifugeant à chaque fois à 48000 xg et en remettant le culot en suspension pendant 10 mn dans du tampon frais refroidi. Finalement on met le dernier culot en suspension dans le tampon pour arriver à une concentration de 100 mg de tissu de départ par ml de tampon à 50 mM. On laisse ensuite incuber à 37°C pendant 10 mn.

La liaison avec la [$^3$H]-8-OH-DPAT est déterminée par incubation de 10 μl de suspension de membranes dans un volume final de 1 ml de tampon contenant 10 μM de pargylline.

Après l'incubation on récupère les membranes par filtration sur filtres Whatman GF/B qu'on lave trois fois avec des quantités aliquotes de 5 ml de tampon glacé. On extrait les filtres dans le liquide de scintillation et on en mesure la radioactivité par scintigraphie liquide. La liaison spécifique de la [$^3$H]-8-OH-DPAT est définie comme la quantité radioactive retenue sur les filtres et pouvant être inhibée par co-incubation dans la hydroxy-5 tryptamine à 10 μM. A une concentration de 1 nM de [$^3$H]-8-OH-DPAT la liaison spécifique représente de 70 à 80% de la radioactivité totale recupérée sur le filtre.

Pour chaque concentration de composés étudiés on détermine le pourcentage d'inhibition de la liaison avec la [$^3$H]-8-OH- DPAT, puis la concentration $CI_{50}$, concentration qui inhibe 50% de la liaison.

Pour les composés de l'invention les $CI_{50}$ se situent entre 0,001 et 0,03 μM.

L'activité centrale des composés de l'invention a été évaluée par leurs effets sur les "pointes PGO" (ponto-géniculo-occipitales) induites par la réserpine (test PGO-R) chez le chat, selon la méthode décrite par H. Depoortere, Sleep 1976, 3rd Europ. Congr. Sleep Res., Montpellier 1976, 358-361 (Karger, Basel 1977).

On administre des doses cumulatives de composés à étudier (de 0,01 à 3 mg/kg par voie intraveineuse) à des intervalles de temps de 30 mn, 4 h après injection intrapéritonéale d'une dose de 0,75 mg/kg de réserpine, à des chats curarisés, sous ventilation artificielle. On recueille les activités électroencéphalographique et phasique (pointes PGO-R) à l'aide d'électrodes corticales et profondes (genouillé latéral). Pour chaque dose de composé étudié on détermine le pourcentage de diminution du nombre de pointes PGO, puis la $DA_{50}$, dose active qui diminue de 50% ce nombre de pointes. Pour les composés de l'invention les $DA_{50}$ se situent entre 0,001 et 0,1 mg/kg par la voie intraveineuse.

Les résultats des essais montrent que certains des composés de formule générale (I) possèdent une grande affinité et une grande sélectivité pour les récepteurs sérotoninergiques de type 5-$HT_{1A}$. In vivo ils montrent une activité soit agoniste, soit agoniste partielle, soit antagoniste au niveau de ces récepteurs.

Certains composés de l'invention possèdent en outre une activité antisérotonine au niveau des récepteurs de type 5HT2.

Cette activité a été mise en évidence "in vitro" par déplacement de ligands fixés spécifiquement sur les récepteurs sérotoninergiques (test de binding SBS) et "in vivo" par antagonisme des effets de la sérotonine au niveau périphérique (test OES) et au niveau central (test AHT).

Test SBS : les composés de l'invention ont fait l'objet d'un essai de déplacement de la liaison (binding) du spiropéridol sur les récepteurs sérotoninergiques (5-HT2) du cortex cérébral du rat.

Pour cet essai on prélève les cerveaux de rats, on en dissèque le cortex et on l'homogénéise à 0°C dans 10 volumes d'un mélange contenant, par litre, 50 millimoles de tampon Tris/HCl à pH = 7,4, 120 millimoles de chlorure de sodium et 5 millimoles de chlorure de potassium. On centrifuge le mélange homogène à 40000 xg pendant 10 mn puis, à deux reprises, on récupère le culot, on le lave en le mettant en suspension dans le même mélange tampon, on l'homogénéise de nouveau et on le centrifuge. Pour terminer on dilue le culot final dans le même mélange tampon à raison de 100 mg de tissu humide pour 1 ml de tampon.

On soumet alors le tissu à une incubation préalable de 10 mn à 37°C en présence de 10 micromoles/l de pargyline, puis à une incubation de 20 mn à 37°C en présence de $^3$H-spiropéridol (activité spécifique : 25,6 Ci par millimole) à la concentration de 0,3 nanomole/l et de composé à étudier à des concentrations allant de 0,0001 à 100 micromoles/l.

On prélève des aliquots de 1 ml que l'on filtre sous vide, on lave les filtres deux fois avec 5 ml de tampon froid et on les sèche. La radioactivité est mesurée dans le toluène en présence de 5 g/l de diphényl-2,5 oxazole (PPO) et 0,1 g/l de bis-(phényl-5 oxazolyl-2)-1,4 benzène (POPOP).

Pour évaluer l'activité des composés on établit la courbe du pourcentage d'inhibition de la liaison spécifique de $^3$H-spiropéridol en fonction de la concentration en drogue déplaçante. On détermine graphiquement la concentration $IC_{50}$, concentration qui inhibe 50% de la liaison spécifique.

La liaison spécifique est définie comme étant la liaison déplacée par 100 micromoles/l de 5-HT.

Les concentrations $IC_{50}$ des composés de l'invention se situent pour la plupart entre 1 et 50 nanomoles/l.

Test OES : l'activité antisérotoninergique des composés de l'invention a également été mise en évidence par leur effet sur l'oedème provoqué chez le rat par la sérotonine, selon la méthode décrite par Maling et coll., J. Pharmacol. Exp. Therap., 191 (2), 300-310 (1974).

Les animaux sont des rats mâles de souche CD (Ch. River, France) pesant 120 à 150 g, à jeun depuis 18 h et répartis en blocs randomisés.

Les composés, mis en solution ou en suspension dans du Tween 80$^R$ à 1%, sont administrés par voie orale

à raison de 0,5 ml pour 100 g de poids corporel, 1 h avant l'injection sous-plantaire, dans l'une des pattes postérieures, de 1 µg de sérotonine (en solution dans du sérum physiologique stérile, sous un volume de 0,1 ml). Le volume de l'oedème est mesuré 1 h après l'injection de sérotonine au moyen d'un pléthysmomètre à mercure Ugo Basile. La $DA_{40}$ (dose qui diminue de 40% le volume de l'oedème, par rapport aux animaux témoins) est déterminée graphiquement.

La $DA_{40}$ des composés de l'invention, déterminée par voie orale est comprise entre 0,1 et 2 mg/kg.

Test AHT : l'activité antisérotoninergique des composés a été étudiée quant à leur effet sur l'antagonisme des "head-twitches" (ébrouements de la tête) provoqués par le L-5-hydroxy-tryptophane (L-5-HTP) chez la souris, selon la méthode décrite par Corne et coll., Br. J. Pharmacol., 20, 106-120 (1962).

Les souris (mâles CD1, Charles River France ; 18-22 g de poids corporel) reçoivent les produits à étudier, à doses croissantes, ou le solvant, par voie intrapéritonéale ou orale, simultanément (voie i.p.) ou soixante minutes avant (voie orale) une injection de L-5-HTP à la dose de 250 mg/kg par voie sous-cutanée. Quarante-cinq minutes après cette injection de 5-HTP, le nombre d'ébrouements est compté, pour chaque souris, pendant une minute.

Pour chaque traitement, la moyenne des ébrouements, ainsi que le pourcentage de variation par rapport au lot témoin, sont calculés.

A partir de la courbe effet-dose, on détermine la $DA_{50}$ (dose active 50% ou dose qui diminue de 50% le nombre moyen d'ébrouements par rapport aux animaux témoins) par la méthode graphique de Miller et Tainter (Proc. Soc. Exp. Biol. Med., (1944), 57, 261).

Les $DA_{50}$ des composés de l'invention se situent entre 0,05 et 2 mg/kg par voie intrapéritonéale et entre 1 et 4 mg/kg par voie orale.

Les composés de l'invention peuvent être utilisés pour le traitement de la migraine, l'anxiété, la dépression, l'obésité, la schizophrénie, les spasmes vasculaires ou gastrointestinaux, l'hypertension et l'agrégation plaquettaire, et comme antiémétiques. Ils peuvent également être utilisés pour le traitment des troubles psychocomportementaux de la sénescence cérébrale.

Les composés de l'invention peuvent être administrés par voie orale ou parentérale, en association avec tout excipient approprié.

La posologie quotidienne peut aller de 1 à 1000 mg.

ANNEXE

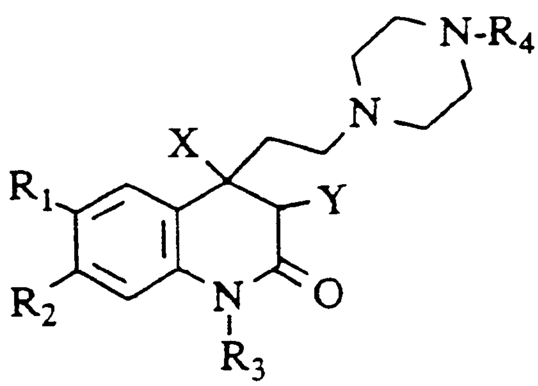

(I)

## ANNEXE 1

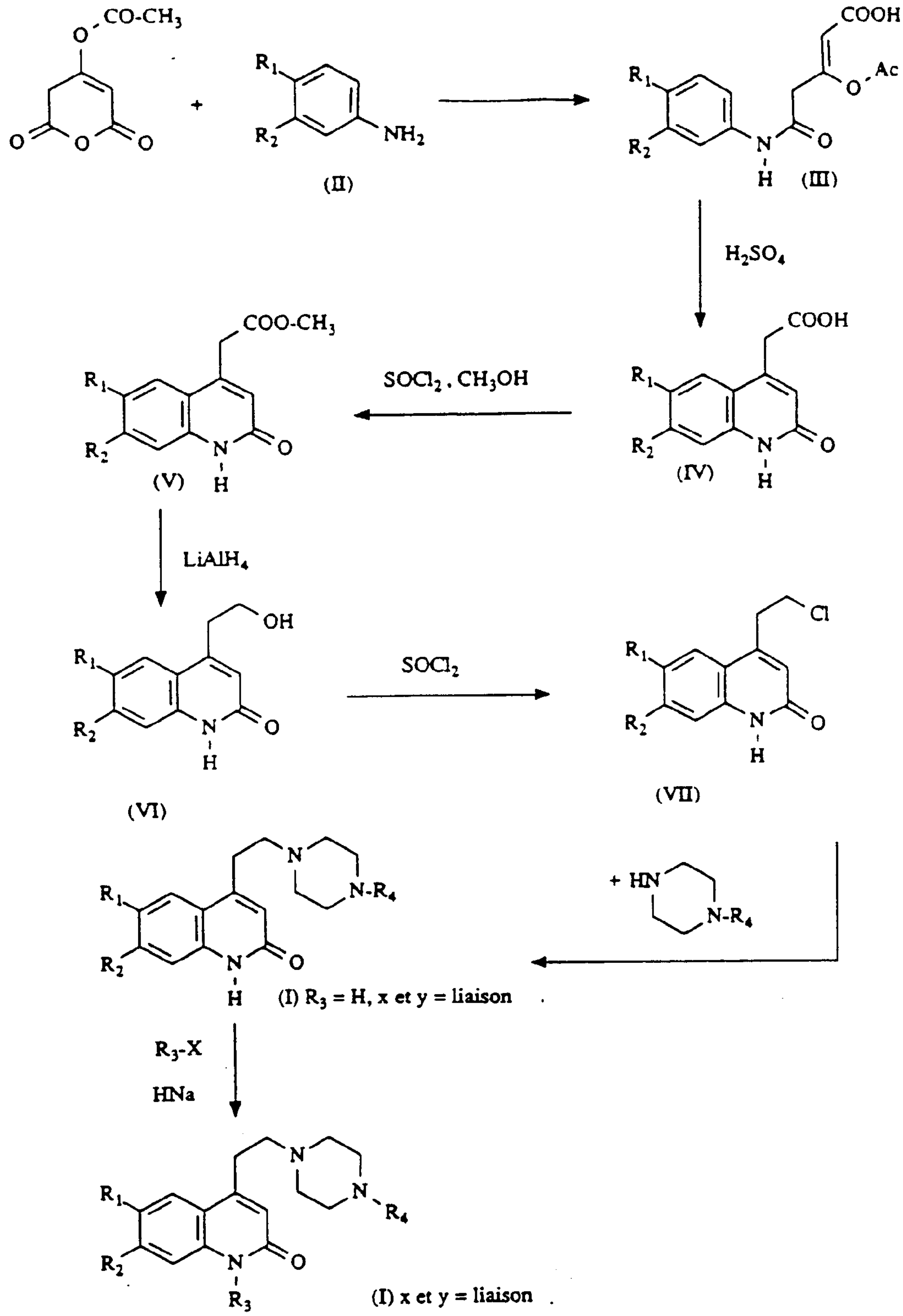

(II)
(III)
$H_2SO_4$
(IV)
$SOCl_2$, $CH_3OH$
(V)
$LiAlH_4$
(VI)
$SOCl_2$
(VII)
(I) $R_3$ = H, x et y = liaison
$R_3$-X
HNa
(I) x et y = liaison

ANNEXE 2

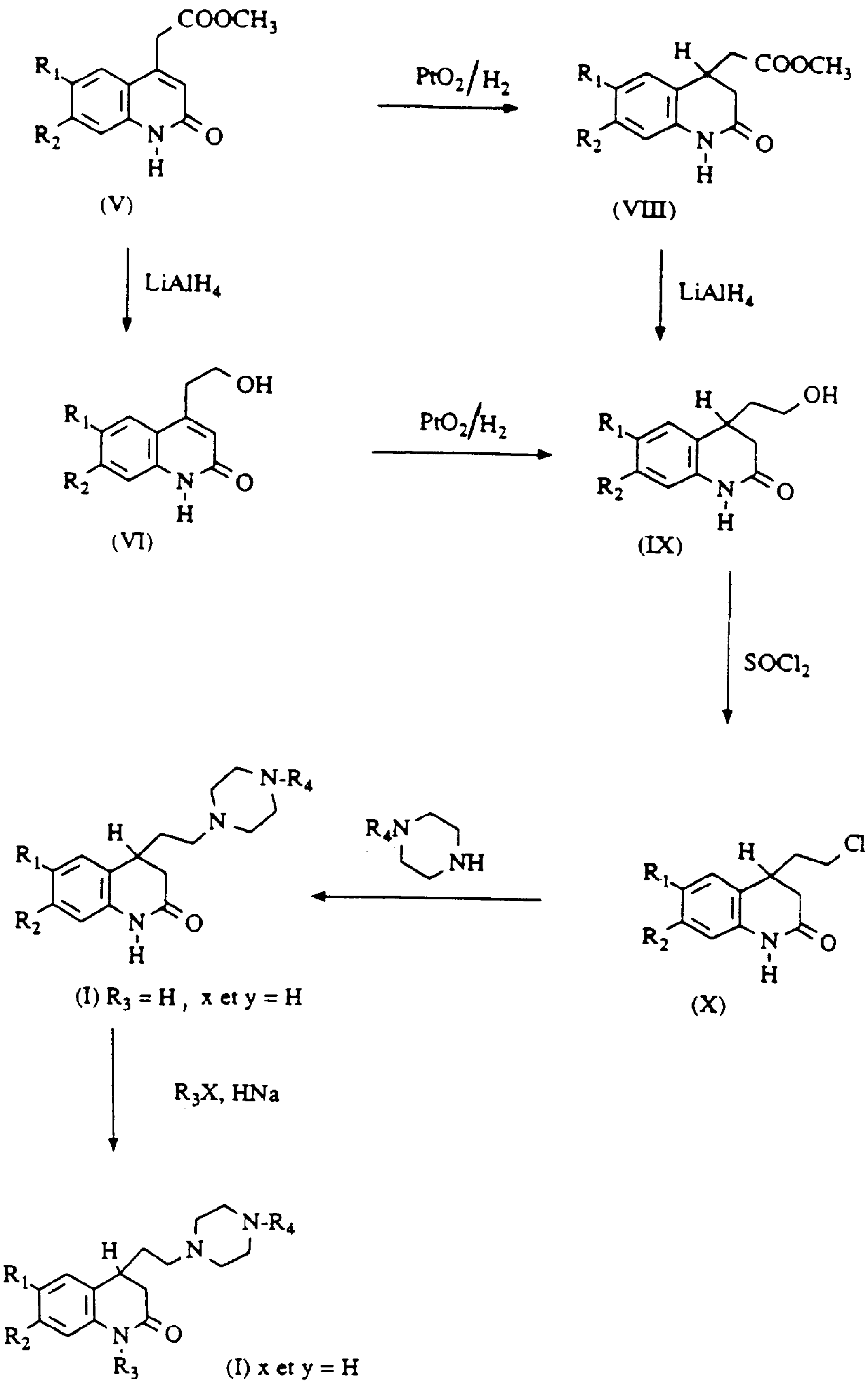


(V)

(VIII)

(VI)

(IX)

(X)

(I) $R_3$ = H , x et y = H

(I) x et y = H

ANNEXE 3

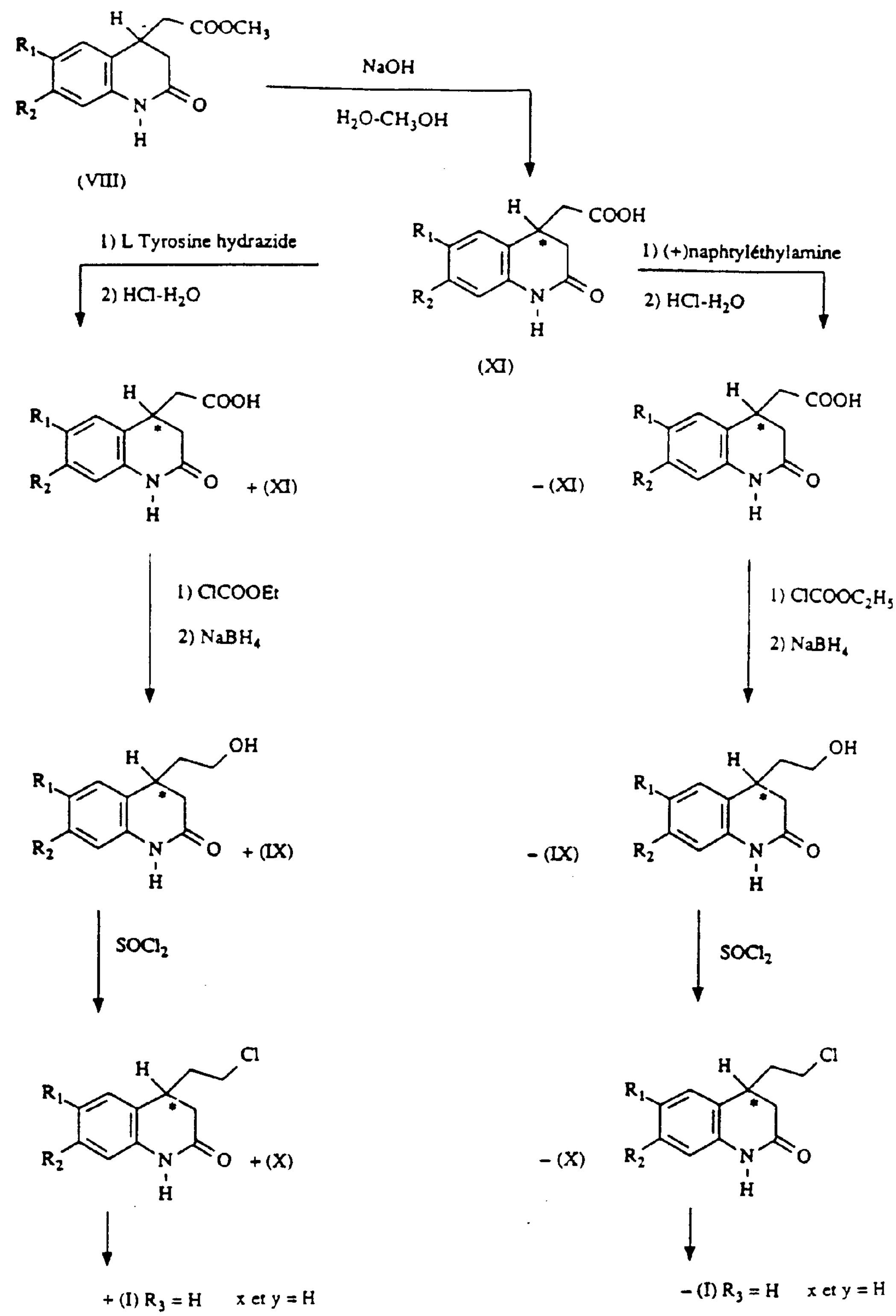

NaOH
$H_2O$-$CH_3OH$
(VIII)
1) L Tyrosine hydrazide
2) HCl-$H_2O$
(XI)
1) (+)naphtyléthylamine
2) HCl-$H_2O$
+ (XI)
– (XI)
1) ClCOOEt
2) $NaBH_4$
1) $ClCOOC_2H_5$
2) $NaBH_4$
+ (IX)
– (IX)
$SOCl_2$
$SOCl_2$
+ (X)
– (X)
+ (I) $R_3$ = H x et y = H
– (I) $R_3$ = H x et y = H

## Revendications

### Revendications pour les Etats Contractants suivants : AT, BE, CH, DE, FR, GB, IT, LI, LU, NL, SE

1. Dérivés de quinoléinone répondant à la formule (I)

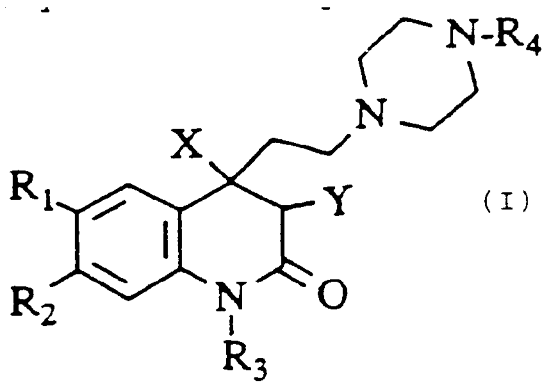

(I)

dans laquelle $R_1$ et $R_2$ sont chacun, indépendamment l'un de l'autre, un atome d'hydrogène ou d'halogène ou un radical $(C_{1-4})$alkyle,

$R_3$ est un atome d'hydrogène ou un radical $(C_{1-4})$alkyle,

$R_4$ est un radical naphtalényle, tétrahydronaphtalényle, benzyle, phényle, pyridyle, isoquinolyle ou benzoyle, ces radicaux pouvant porter un substituant tel que méthoxy, chlore, fluor ou $(C_{3-5})$ cycloalkyle,

X et Y sont chacun un atome d'hydrogène ou forment ensemble une liaison,

ainsi que leurs sels d'addition aux acides pharmaceutiquement acceptables et, lorsque X et Y sont chacun un atome d'hydrogène, les diastéréoisomères et énantiomères des composés.

2. Dérivés de quinoléinone selon la revendication 1, caractérisés par le fait que $R_1$ et $R_2$ sont chacun indépendamment l'un de l'autre, un atome d'hydrogène ou de fluor ou le radical méthyle,

$R_3$ est un atome d'hydrogène ou le radical méthyle ou éthyle,

$R_4$ est le radical naphtalényle ou tétrahydronaphtalényle, éventuellement substitué, et

X et Y représentent une liaison ou chacun un atome d'hydrogène.

3. La (±) [[(méthoxy-7 naphtalényl-1)-4 pipérazinyl-1]-2 éthyl]-4 dihydro-3,4 1H-quinoléinone-2, ses sels et ses énantiomères.

4. La (±)[[(méthoxy-7 naphtalényl-1)-4 pipérazinyl-1]-2 éthyl]-4 méthyl-6 dihydro-3,4 1H-quinoléinone-2, ses sels et ses énantionmères.

5. La [[(naphtalényl-1)-4pipérazinyl-1]-2 éthyl]-4 1H-quinoléinone-2 et ses sels.

6. Procédé de préparation des composés (I) dans lesquels X et Y représentent ensemble une liaison, procédé caractérisé en ce que l'on fait réagir une phénylamine (II)

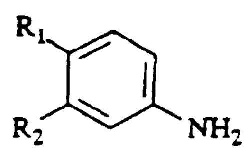

avec l'(acétyloxy)-4 2H,3H-pyrannedione-2,6 et on cyclise le composé obtenu (III)

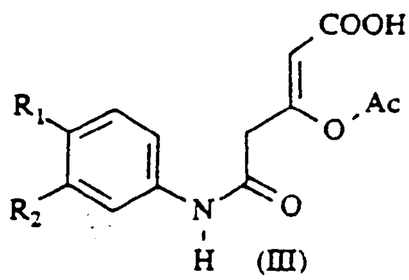

(III)

en acide (IV)

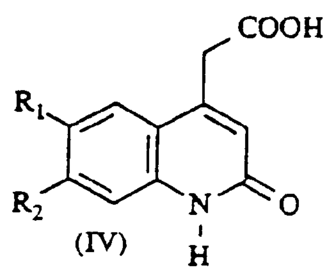

(IV)

que l'on transforme en ester (V)

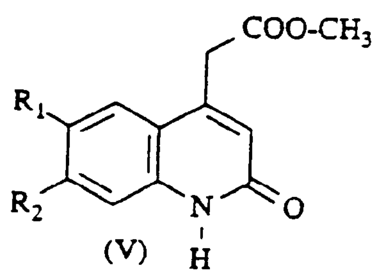

(V)

puis en alcool (VI)

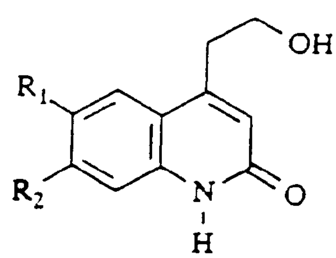

(VI)

et enfin en chlorure (VII)

(VII)

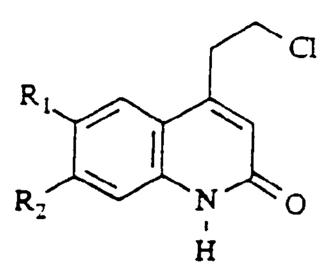

que l'on condense avec une $R_4$-1 pipérazine pour obtenir le composé (I), que l'on alkyle, si on le souhaite, avec un composé $R_3X$ (X = groupe labile).

7. Procédé de préparation des composés (I) dans lesquels X et Y représentent chacun un atome d'hydrogène, procédé caractérisé en ce que l'on hydrogène l'ester (V) en ester (VIII)

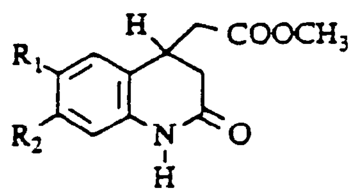

(VIII)

que l'on réduit en alcool (IX)

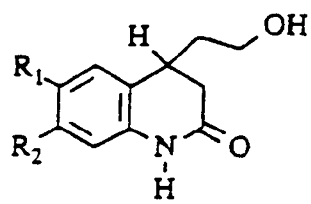

(IX)

lequel est transformé en chlorure (X)

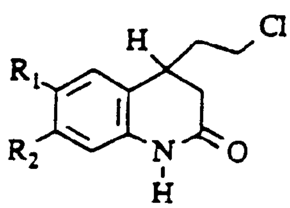

(X)

que l'on condense avec une $R_4$-1 pipérazine pour obtenir le composé (I) (X,Y = H,H) que l'on alkyle, si on le souhaite, avec un composé $R_3X$ (X = groupe labile).

8. Procédé de préparation des composés (I) dans lesquels X et Y représentent chacun un atome d'hydrogène, procédé caracterisé en ce que l'on hydrogène l'alcool (VI) en alcool (IX)

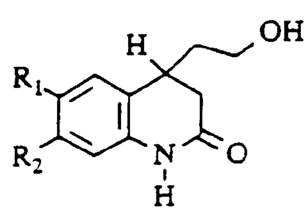

(IX)

et l'on poursuit la synthèse selon la revendication 7.

9. Procédé de préparation des énantiomères des composés (I) dans lesquels X et Y sont des atomes d'hydrogène, procédé caractérisé en ce que l'on saponifie l'ester (VIII) en acide (XI)

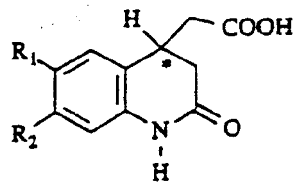

(XI)

que l'on dédouble en ses deux énantiomères à l'aide d'une amine optiquement active telle que la L-tyrosine-hydrazide ou l'un des deux énantiomères de la (naphtyl-1)-1 éthylamine, ces deux acides (+)(XI) et (–)(XI) étant ensuite réduits en alcools (+)(IX) et (–)(IX)

(IX) 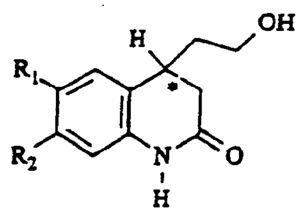

lesquels sont alors transformés en dérivés chlorés (+) (X) et (–) (X)

(X) 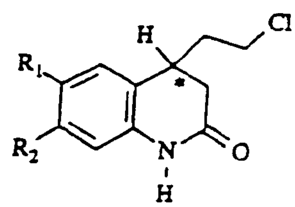

que l'on condense avec une $R_4$-1 pipérazine pour obtenir les composés (+) (I) et (–) (I) (X,Y = H,H) que l'on alkyle, si on le souhaite, avec un composé $R_3X$ (X = groupe labile).

10. Médicament caractérisé en ce qu'il contient un composé tel que spécifié dans l'une des revendications 1 à 5.

11. Composition pharmaceutique caracterisée en ce qu'elle contient un composé tel que spécifié dans l'une des revendications 1 à 5, en association avec tout excipient approprié.

**Revendications pour les Etats contractants suivants : ES, GR**

1. Procédé de préparation de dérivés de quinoléinone répondant à la formule (I)

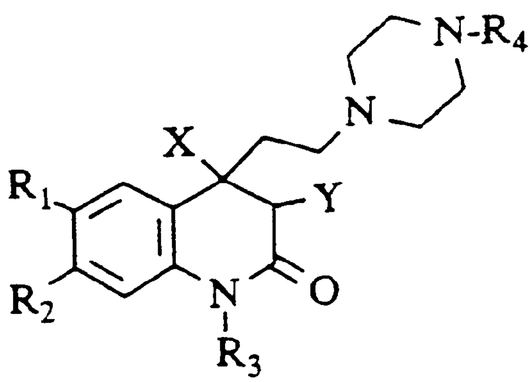

(I)

dans laquelle $R_1$ et $R_2$ sont chacun, indépendamment l'un de l'autre, un atome d'hydrogène ou d'halogène ou

un radical ($C_{1-4}$)alkyle,

$R_3$ est un atome d'hydrogène ou un radical ($C_{1-4}$)alkyle,

$R_4$ est un radical naphtalényle, tétrahydronaphtalényle, benzyle, phényle, pyridyle, isoquinolyle ou benzoyle, ces radicaux pouvant porter un substituant tel que méthoxy, chlore, fluor ou ($C_{3-5}$) cycloalkyle,

X et Y sont chacun un atome d'hydrogène ou forment ensemble une liaison,

ainsi que de leurs sels d'addition aux acides pharmaceutiquement acceptables et, lorsque X et Y sont chacun un atome d'hydrogène, des diastéréoisomères et énantiomères des composés, procédé caractérisé en ce que

a) pour préparer les composés dans lesquels X et Y représentent ensemble une liaison, on fait réagir une phénylamine (II)

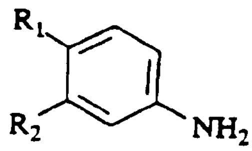

avec l'(acétyloxy)-4 2H,3H-pyrannedione-2,6 et on cyclise le composé obtenu (III)

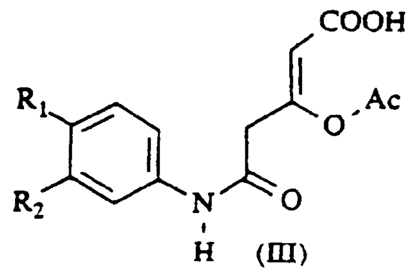

(III)

en acide (IV)

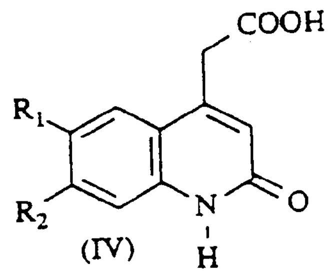

(IV)

que l'on transforme en ester (V)

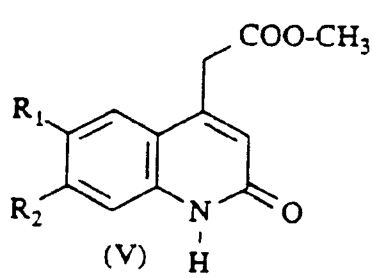

(V)

puis en alcool (VI)

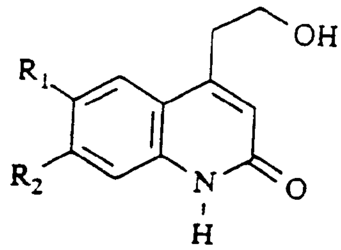

(VI)

et enfin en chlorure (VII)

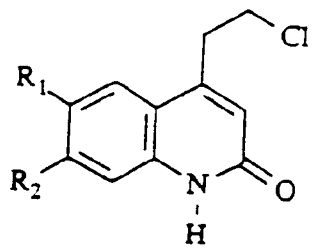

(VII)

que l'on condense avec une $R_4$-1 pipérazine pour obtenir le composé (I), que l'on alkyle, si on le souhaite, avec un composé $R_3X$ (X = groupe labile),

b) pour préparer les composés dans lesquels X et Y représentent chacun un atome d'hydrogène, on hydrogène l'ester (V) en ester (VIII)

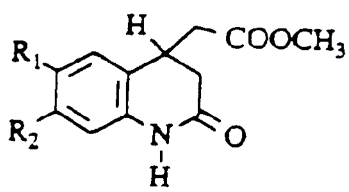

(VIII)

que l'on réduit en alcool (IX)

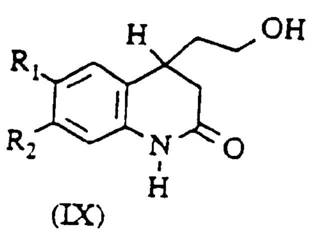

(IX)

lequel est transformé en chlorure (X)

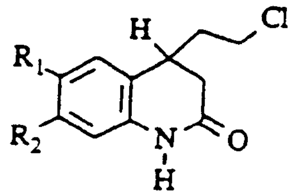

(X)

que l'on condense avec une $R_4$-1 pipérazine pour obtenir le composé (I) (X,Y = H,H) que l'on alkyle, si on le souhaite, avec un composé $R_3X$ (X = groupe labile).

2. Procédé de préparation des énantiomères des composés (I) dans lesquels X et Y sont des atomes d'hydrogène, procédé caractérisé en ce que l'on saponifie l'ester (VIII) en acide (XI)

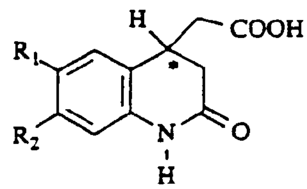

(XI)

que l'on dédouble en ses deux énantiomères à l'aide d'une amine optiquement active telle que la L-tyrosine-hydrazide ou l'un des deux énantiomères de la (naphtyl-1)-1 éthylamine, ces deux acides (+)(XI) et (–)(XI) étant ensuite réduits en alcools (+)(IX) et (–)(IX)

(IX) 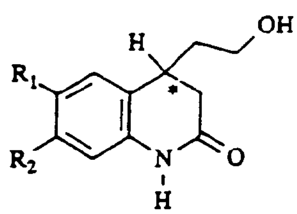

lesquels sont alors transformés en dérivés chlorés (+)(X) et (–)(X)

(X) 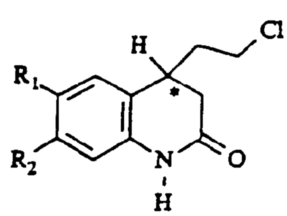

que l'on condense avec une $R_4$-1 pipérazine pour obtenir les composés (+)(I) et (–)(I) (X,Y = H,H) que l'on alkyle, si on le souhaite, avec un composé $R_3X$ (X = groupe labile).

3. Procédé de préparation des composés selon la revendication 1, procédé caractérisé en ce que l'on prépare les composés dans lesquels $R_1$ et $R_2$ sont chacun indépendamment l'un de l'autre, un atome d'hydrogène ou de fluor ou le radical méthyle,
$R_3$ est un atome d'hydrogène ou le radical méthyle ou éthyle,
$R_4$ est le radical naphtalényle ou tétrahydronaphtalényle, éventuellement substitué, et
X et Y représentent une liaison ou chacun un atome d'hydrogène.

## Patentansprüche

## Patentansprüche für folgende Vertragsstaaten : AT, BE, CH, DE, FR, GB, IT, LI, LU, NL, SE

1. Chinolinonderivate der Formel (I)

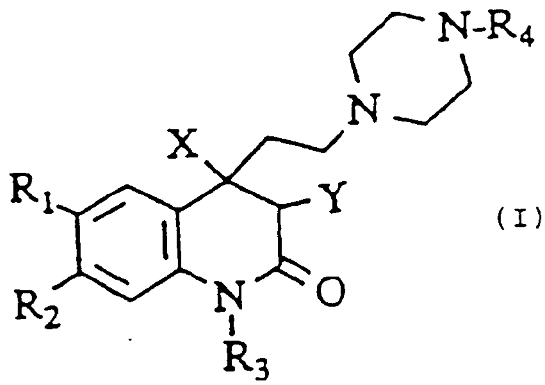

(I)

in welcher $R_1$ und $R_2$ jeweils unabhängig voneinander für ein Wasserstoff- oder ein Halogenatom oder einen $(C_{1-4})$-Alkylrest stehen,

$R_3$ ein Wasserstoffatom oder einen $(C_{1-4})$-Alkylrest darstellt,

$R_4$ einen Naphthalinyl-, Tetrahydronaphthalinyl-, Benzyl-, Phenyl-, Pyridyl-, Isochinolyl- oder Benzoylrest bedeutet, welche Reste einen Substituenten, wie Methoxy, Chlor, Fluor oder $(C_{3-5})$-Cycloalkyl, tragen können, und

X und Y jeweils ein Wasserstoffatom darstellen oder gemeinsam eine Bindung bilden,

sowie deren Additionssalze mit pharmazeutisch verwendbaren Säuren, und, wenn X und Y jeweils für ein Wasserstoffatom stehen, die Diastereoisomeren und Enantiomeren dieser Verbindungen.

2. Chinolinonderivate nach Anspruch 1, **dadurch gekennzeichnet,** daß $R_1$ und $R_2$ jeweils unabhängig voneinander für ein Wasserstoff- oder Fluoratom oder für den Methylrest stehen,

$R_3$ ein Wasserstoffatom oder den Methyl- oder Ethylrest bedeutet,

$R_4$ einen gegebenenfalls substituierten Naphthalinyl- oder Tetranaphthalinylrest darstellt und

X und Y für eine Bindung stehen oder jeweils ein Wasserstoffatom bedeuten.

3. (±)-4-[2-[4-(7-Methoxy-naphthalinyl-1)-piperazinyl-1]-ethyl]-3,4-dihydro-1H-chinolinon-2, seine Salze und seine Enantiomeren.

4. (±)-4-[2-[4-(7-Methoxy-naphthalinyl-1)-piperazinyl-1)-ethyl]-6-methyl-3,4-dihydro-1H-chinolinon-2, seine Salze und seine Enantiomeren.

5. 4-[2-[4-(Naphthalinyl-1)-piperazinyl-1]-ethyl]-1H-chinolinon-2 und seine Salze.

6. Verfahren zur Herstellung der Verbindungen (I), in welchen X und Y gemeinsam für eine Bindung stehen, **dadurch gekennzeichnet,** daß man ein Phenylamin (II)

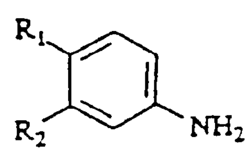

mit 4-Acetyloxy-2,6-2H,3H-pyrandion reagieren läßt und die erhaltene Verbindung (III)

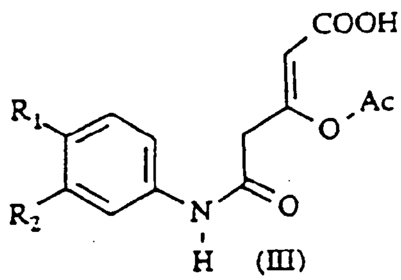

(III)

zur Säure (IV)

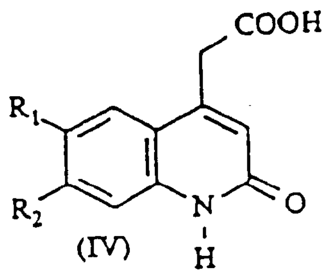

(IV)

zyklisiert, worauf man diese in den Ester (V)

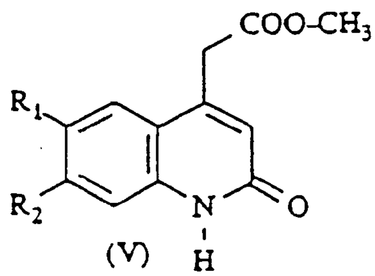

(V)

dann in den Alkohol (VI)

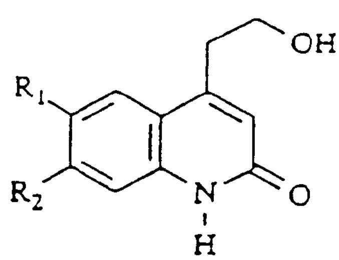

(VI)

und schließlich in das Chlorid (VII)

(VII)

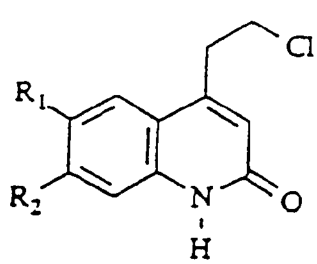

umwandelt, das man mit einem 1-$R_4$-Piperazin kondensiert, um die Verbindung (I) zu erhalten, die man gewünschtenfalls mit einer Verbindung $R_3X$ (X = eine labile Gruppe) alkyliert.

7. Verfahren zur Herstellung der Verbindungen (I), in welchen X und Y jeweils für ein Wasserstoffatom stehen, **dadurch gekennzeichnet,** daß man den Ester (V) zum Ester (VIII)

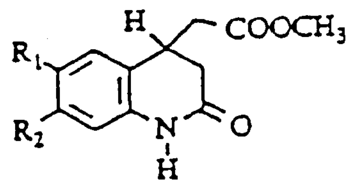

(VIII)

hydriert, worauf man diesen zum Alkohol (IX)

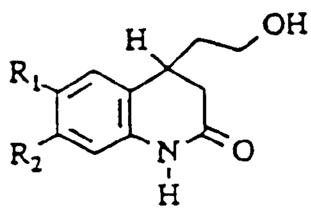

(IX)

reduziert, welchen man in das Chlorid (X)

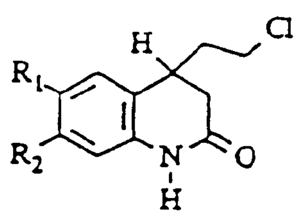

(X)

umwandelt, worauf man dieses mit mit einem 1-$R_4$-Piperazin kondensiert, um die Verbindung (I) (X,Y = H,H) zu erhalten, die man gewünschtenfalls mit einer Verbindung $R_3X$ (X = labile Gruppe) alkyliert.

8. Verfahren zur Herstellung der Verbindungen (I), in welchen X und Y jeweils für ein Wasserstoffatom stehen, **dadurch gekennzeichnet,** daß man den Alkohol (VI) zum Alkohol (IX)

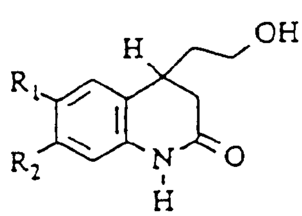

(IX)

hydriert und dann die Synthese nach Anspruch 7 fortführt.

9. Verfahren zur Herstellung der Enantiomeren der Verbindungen (I), in welchen X und Y Wasserstoffatome darstellen, **dadurch gekennzeichnet,** daß man den Ester (VIII) zur Säure (XI)

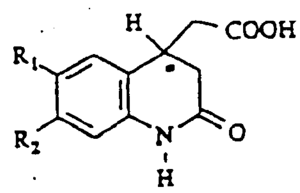

(XI)

verseift, diese mit Hilfe eines optisch aktiven Amins, wie L-Tyrosinhydrazid oder eines der beiden Enantiomeren des 1-(1-Naphthyl)-ethylamins, in seine beiden Enantiomeren aufspaltet, worauf diese beiden Säuren (+)(XI) und (–)(XI) anschließend zu den Alkoholen (+)(IX) und (–)(IX)

(IX) 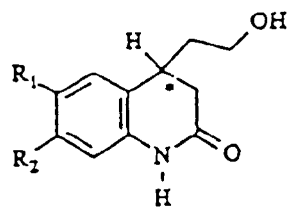

reduziert werden, welch letztere dann in die chlorierten Derivate (+)(X) und (–)(X)

(X) 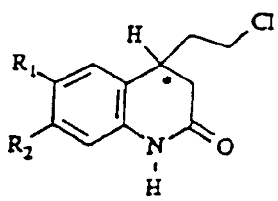

umgewandelt werden, die man mit einem 1-$R_4$-Piperazin kondensiert, um die Verbindungen (+)(I) und (–)(I) (X,Y = H,H) zu erhalten, die dann gewünschtenfalls mit einer Verbindung $R_3X$ (X = labile Gruppe) alkyliert werden.

10. Arzneimittel, **dadurch gekennzeichnet,** daß es eine Verbindung nach einem der Ansprüche 1 bis 5 enthält.

11. Pharmazeutische Zusammensetzung, **dadurch gekennzeichnet,** daß sie eine Verbindung nach einem der Ansprüche 1 bis 5 in Kombination mit irgendeinem geeigneten Trägermittel enthält.

**Patentansprüche für folgende Vertragsstaaten : ES, GR**

1. Verfahren zur Herstellung von Chinolinonderivaten der Formel (I)

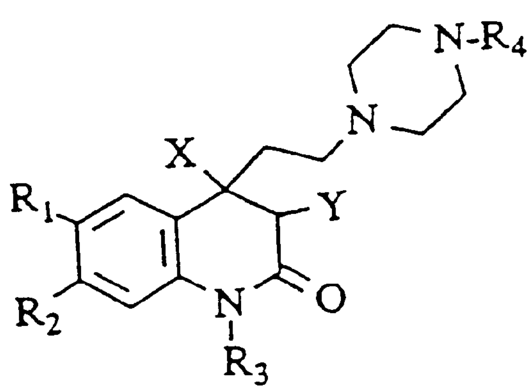

(I)

in welcher $R_1$ und $R_2$ jeweils unabhängig voneinander für ein Wasserstoff- oder ein Halogenatom oder einen

($C_{1-4}$)-Alkylrest stehen,

$R_3$ ein Wasserstoffatom oder einen ($C_{1-4}$)-Alkylrest darstellt,

$R_4$ einen Naphthalinyl-, Tetrahydronaphthalinyl-, Benzyl-, Phenyl-, Pyridyl-, Isochinolyl- oder Benzoylrest bedeutet, welche Reste einen Substituenten, wie Methoxy, Chlor, Fluor oder ($C_{3-5}$)-Cycloalkyl, tragen können, und

X und Y jeweils ein Wasserstoffatom darstellen oder gemeinsam eine Bindung bilden,

sowie ihren Additionssalzen mit pharmazeutisch verwendbaren Säuren, und, wenn X und Y jeweils für ein Wasserstoffatom stehen, den Diastereoisomeren und Enantiomeren dieser Verbindungen, **dadurch gekennzeichnet,** daß man

a) zur Herstellung von Verbindungen, in welchen X und Y gemeinsam für eine Bindung stehen, ein Phenylamin (II)

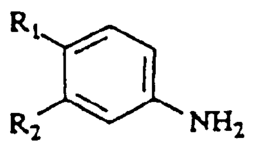

mit 4-Acetyloxy-2,6-2H,3H-pyrandion reagieren läßt und die erhaltene Verbindung (III)

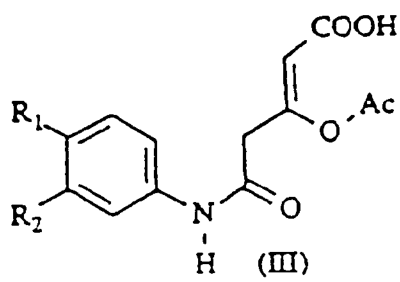

(III)

zur Säure (IV)

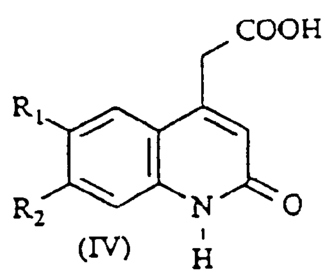

(IV)

zyklisiert, worauf man diese in den Ester (V)

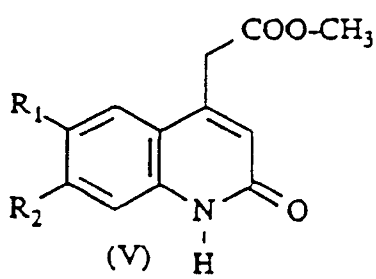

(V)

dann in den Alkohol (VI)

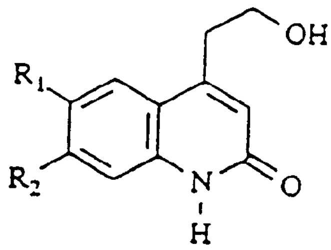

(VI)

und schließlich in das Chlorid (VII)

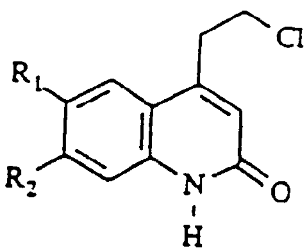

(VII)

umwandelt, das man mit einem 1-$R_4$-Piperazin kondensiert, um die Verbindung (I) zu erhalten, die man gewünschtenfalls mit einer Verbindung $R_3X$ (X = eine labile Gruppe) alkyliert,

b) zur Herstellung von Verbindungen, in denen X und Y jeweils für ein Wasserstoffatom stehen, den Ester (V) zum Ester (VIII)

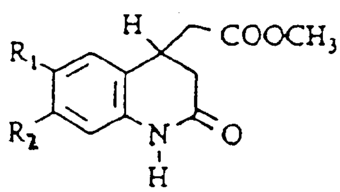

(VIII)

hydriert, worauf man diesen zum Alkohol (IX)

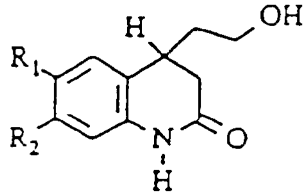

(IX)

reduziert, welchen man in das Chlorid (X)

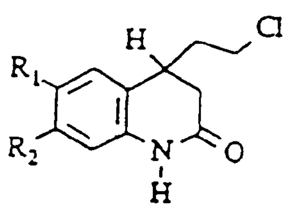

(X)

umwandelt, das man mit mit einem 1-$R_4$-Piperazin kondensiert, um die Verbindung (I) (X,Y = H,H) zu erhalten, die man gewünschtenfalls mit einer Verbindung $R_3X$ (X = labile Gruppe) alkyliert.

2. Verfahren zur Herstellung der Enantiomeren der Verbindungen (I), in welchen X und Y für ein Wasserstofftom stehen, **dadurch gekennzeichnet,** daß man den Ester (VIII) zur Säure (XI)

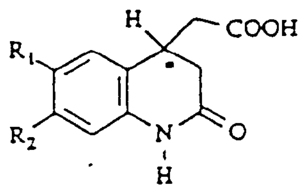

(XI)

verseift, diese mit Hilfe eines optisch aktiven Amins, wie L-Tyrosinhydrazid oder eines der beiden Enantiomeren des 1-(1-Naphthyl)-ethylamins, in seine beiden Enantiomeren aufspaltet, worauf diese beiden Säuren (+)(XI) und (–)(XI) anschließend zu den Alkoholen (+)(IX) und (–)(IX)

(IX) 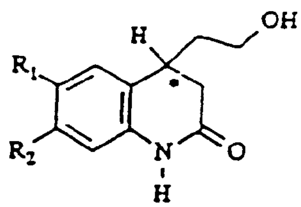

reduziert werden, welch letztere dann in die chlorierten Derivate (+)(X) und (–)(X)

(X) 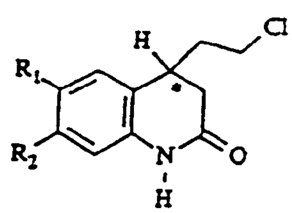

umgewandelt werden, die man mit einem 1-$R_4$-Piperazin kondensiert, um die Verbindungen (+)(I) und (–)(I) (X,Y = H,H) zu erhalten, die dann gewünschtenfalls mit einer Verbindung $R_3X$ (X = labile Gruppe) alkyliert werden.

3. Verfahren zur Herstellung von Verbindungen nach Anspruch 1, **dadurch gekennzeichnet,** daß man die Verbindungen herstellt, in welchen $R_1$ und $R_2$ jeweils unabhängig voneinander für ein Wasserstoff- oder Fluoratom oder für den Methylrest stehen,

$R_3$ ein Wasserstoffatom oder den Methyl- oder Ethylrest bedeutet,

$R_4$ einen gegebenenfalls substituierten Naphthalinyl- oder Tetranaphthalinylrest darstellt und

X und Y für eine Bindung stehen oder jeweils ein Wasserstoffatom bedeuten.

## Claims

### Claims for the following Contracting States : AT, BE, CH, DE, FR, GB, IT, LI, LU, NL, SE

1. Quinolinone derivatives corresponding to the formula (I)

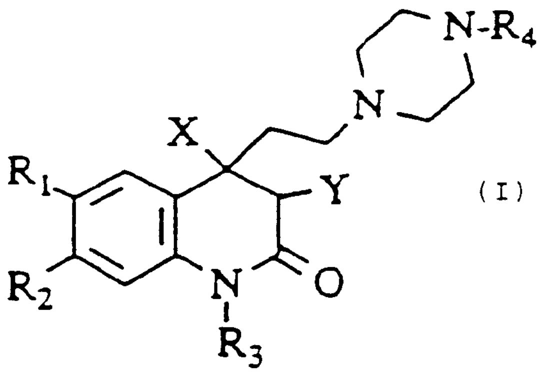

(I)

in which $R_1$ and $R_2$ are each, independently of one another, a hydrogen or halogen atom or a $(C_{1-4})$alkyl radical,

$R_3$ is a hydrogen atom or a $(C_{1-4})$alkyl radical,

$R_4$ is a naphthyl, tetrahydronaphthyl, benzyl, phenyl, pyridyl, isoquinolyl or benzoyl radical, it being possible for these radicals to bear a substituent such as methoxy, chlorine, fluorine or $(C_{3-5})$cycloalkyl, and

X and Y are each a hydrogen atom or together form a bond, as well as their addition salts with pharmaceutically acceptable acids and, when X and Y are each a hydrogen atom, the diastereoisomers and enantiomers of the compounds.

2. Quinolinone derivatives according to Claim 1, characterised in that $R_1$ and $R_2$ are each, independently of one another, a hydrogen or fluorine atom or a methyl radical,

$R_3$ is a hydrogen atom or a methyl or ethyl radical,

$R_4$ is a naphthyl or tetrahydronaphthyl radical, optionally substituted, and

X and Y denote a bond, or each denotes a hydrogen atom.

3. (±)-4-{2-[4-(7-Methoxy-1-naphthyl)-1-piperazinyl]ethyl}-3,4-dihydro-2(1H)-quinolinone, its salts and its enantiomers.

4. (±)-4-{2-[4-(7-Methoxy-1-naphthyl)-1-piperazinyl]ethyl}-6-methyl-3,4-dihydro-2(1H)-quinolinone, its salts and its enantiomers.

5. 4-{2-[4-(1-Naphthyl)-1-piperazinyl]ethyl}-2(1H)-quinolinone and its salts.

6. Process for preparing the compounds (I) in which X and Y together denote a bond, which process is characterised in that a phenylamine (II)

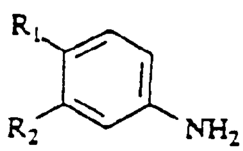

is reacted with 4-acetyloxy-2H,3H-pyran-2,6-dione, and the compound obtained (III)

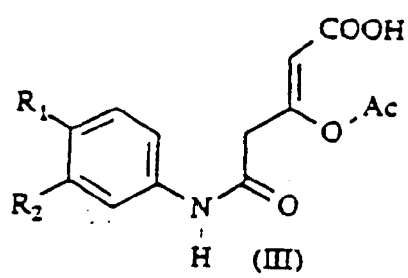

(III)

is cyclised to an acid (IV)

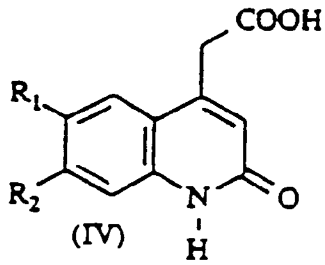

(IV)

which in converted to an enter (V)

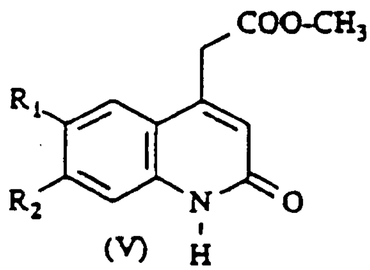

(V)

then to an alcohol (VI)

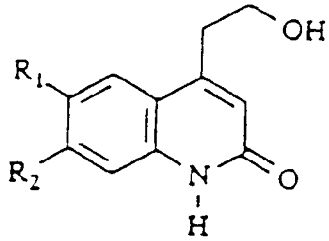

(VI)

and finally to a chloride (VII)

(VII)

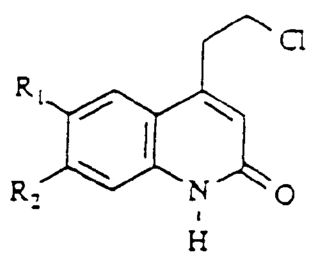

which is condensed with a 1-$R_4$-piperazine to obtain the compound (I), which is alkylated, if so desired, with a compound $R_3X$ (X = labile group).

7. Process for preparing the compounds (I) in which X and Y each denote a hydrogen atom, which process is characterised in that the ester (V) is hydrogenated to an ester (VIII)

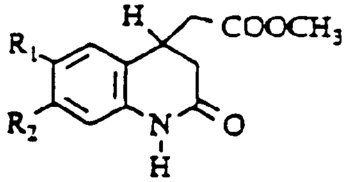

(VIII)

which is reduced to an alcohol (IX)

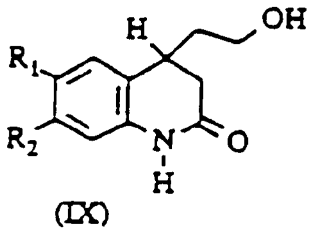

(IX)

which is converted to a chloride (X)

(X)

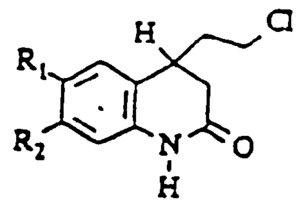

which is condensed with a 1-$R_4$-piperazine to obtain the compound (I) (X,Y = H,H), which is alkylated, if so desired, with a compound R,X (X = labile group).

8. Process for preparing the compounds (I) in which X and Y each denote a hydrogen atom, which process is characterised in that the alcohol (VI) is hydrogenated to an alcohol (IX)

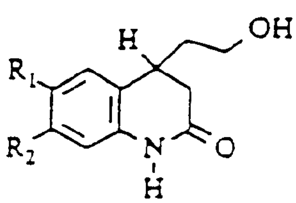

(IX)

and the synthesis is continued according to Claim 7.

9. Process for preparing the enantiomers of the compounds (I) in which X and Y are hydrogen atoms, which process is characterised in that the ester (VIII) is saponified to an acid (XI)

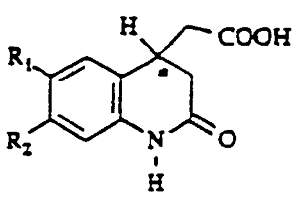

(XI)

which is resolved into its two enantiomers by means of an optically active amine such as L-tyrosine hydrazide or one of the two enantiomers of 1-(1-naphthyl)ethyl-amine, these two acids (+)-(XI) and (–)-(XI) then being

reduced to alcohols (+)-(IX) and (–)-(IX)

(IX) 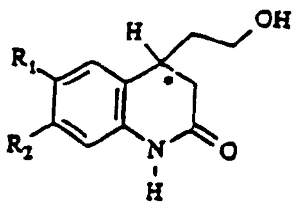

which are then converted to chlorinated derivatives (+)-(X) and (–)-(X)

(X) 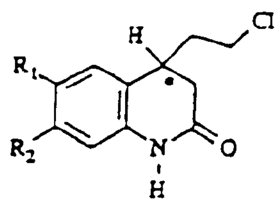

which are condensed with a 1-$R_4$-piperazine to obtain the compounds (+)-(I) and (–)-(I) (X,Y = H,H), which are alkylated, if so desired, with a compound $R_3X$ (X = labile group).

10. Medicinal product, characterised in that it contains a compound as specified in one of Claims 1 to 5.

11. Pharmaceutical composition, characterised in that it contains a compound as specified in one of Claims 1 to 5, in combination with any suitable excipient.

**Claims for the following Contracting States : ES, GR**

1. Process for preparing quinolinone derivatives corresponding to the formula (I)

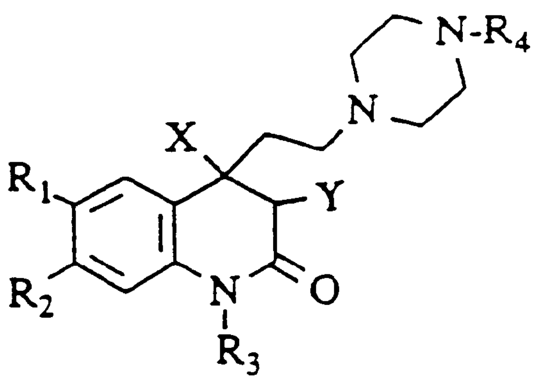 (I)

in which $R_1$ and $R_2$ are each, independently of one another, a hydrogen or halogen atom or a $(C_{1-4})$alkyl radical,
$R_3$ is a hydrogen atom or a $(C_{1-4})$alkyl radical,
$R_4$ is a naphthyl, tetrahydronaphthyl, benzyl, phenyl, pyridyl, isoquinolyl or benzoyl radical, it being possible for these radicals to bear a substituent such as methoxy, chlorine, fluorine or $(C_{3-5})$cycloalkyl, and
X and Y are each a hydrogen atom or together form a bond, as well as their addition salts with pharmaceutically acceptable acids and, when X and Y are each a hydrogen atom, the diastereoisomers and enantiomers of the compounds, which process is characterised in that
a) to prepare the compounds in which X and Y together denote a bond, a phenylamine (II)

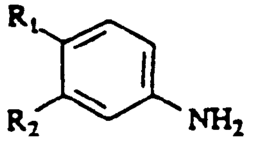

is reacted with 4-acetyloxy-2H,3H-pyran-2,6-dione, and the compound obtained (III)

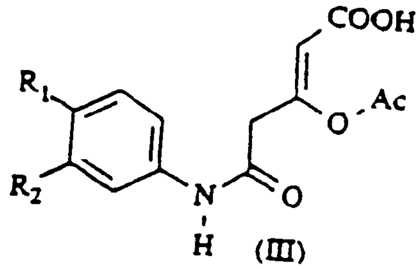

(III)

is cyclised to an acid (IV)

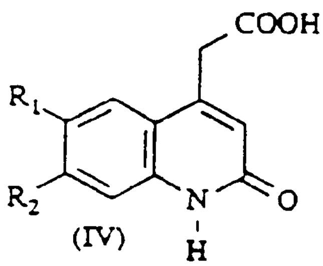

(IV)

which is converted to an ester (V)

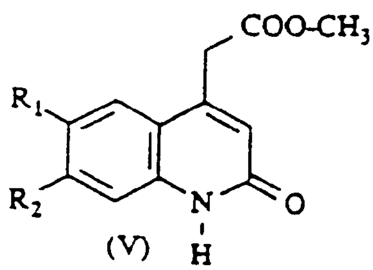

(V)

then to an alcohol (VI)

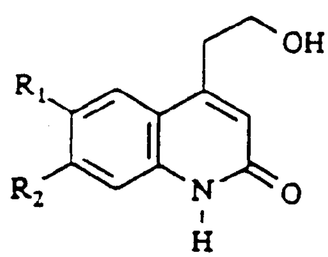

(VI)

and finally to a chloride (VII)

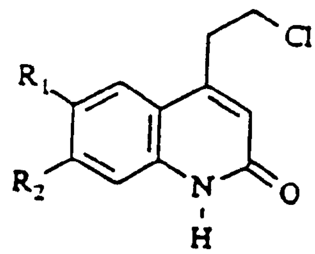

(VII)

which is condensed with a 1-$R_4$-piperazine to obtain the compound (I), which is alkylated, if so desired, with a compound $R_3X$ (X = labile group).

b) to prepare the compounds in which X and Y each denote a hydrogen atom, the ester (V) is hydrogenated to an ester (VIII)

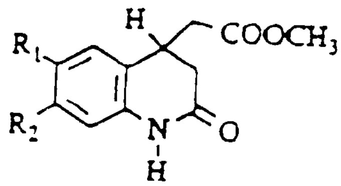

(VIII)

which is reduced to an alcohol (IX)

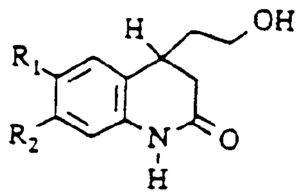

(IX)

which is converted to a chloride (X)

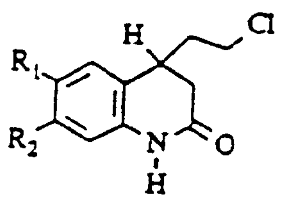

(X)

which is condensed with a 1-$R_4$-piperazine to obtain the compound (I) (X,Y = H,H), which is alkylated, if so desired, with a compound $R_3X$ (X = labile group).

2. Process for preparing the enantiomers of the compounds (I) in which X and Y are hydrogen atoms, which process is characterised in that the ester (VIII) is saponified to an acid (XI)

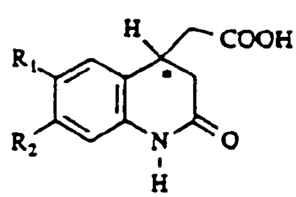

(XI)

which is resolved into its two enantiomers by means of an optically active amine such as L-tyrosine hydrazide or one of the two enantiomers of 1-(1-naphthyl)ethyl-amine, these two acids (+)-(XI) and (–)-(XI) then being reduced to alcohols (+)-(IX) and (–)-(IX)

(IX) 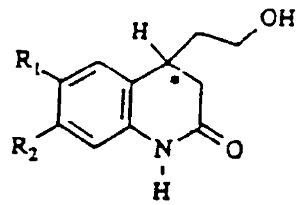

which are then converted to chlorinated derivatives (+)-(X) and (–)-(X)

(X) 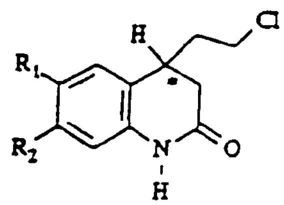

which are condensed with a 1-$R_4$-piperazine to obtain the compounds (+)-(I) and (–)-(I) (X,Y = H,H), which are alkylated, if so desired, with a compound $R_3X$ (X = labile group).

3. Process for preparing the compounds according to Claim 1, which process is characterised in that the compounds in which $R_1$ and $R_2$ are each, independently of one another, a hydrogen or fluorine atom or a methyl radical,

$R_3$ is a hydrogen atom or a methyl or ethyl radical,

$R_4$ is a naphthyl or tetrahydronaphthyl radical, optionally substituted, and

X and Y denote a bond, or each denotes a hydrogen atom, are prepared.